(19) Europäisches Patentamt / European Patent Office / Office européen des brevets

(11) **EP 4 032 854 A1**

(12) **EUROPEAN PATENT APPLICATION**
published in accordance with Art. 153(4) EPC

(43) Date of publication:
**27.07.2022 Bulletin 2022/30**

(21) Application number: **20866362.5**

(22) Date of filing: **08.09.2020**

(51) International Patent Classification (IPC):
*C01B 33/12* $^{(2006.01)}$     *B03C 1/00* $^{(2006.01)}$
*B03C 1/01* $^{(2006.01)}$     *C01B 33/18* $^{(2006.01)}$
*C12N 15/10* $^{(2006.01)}$     *G01N 33/552* $^{(2006.01)}$

(52) Cooperative Patent Classification (CPC):
**B03C 1/288; B03C 1/01; C01B 33/18; C01G 49/08;
C12N 15/1013; H01F 1/0063; H01F 1/36;**
B03C 2201/18; B03C 2201/26; C01P 2004/51;
C01P 2004/60; C01P 2004/61; C01P 2004/62;
C01P 2004/64; C01P 2004/80;      (Cont.)

(86) International application number:
**PCT/JP2020/034009**

(87) International publication number:
**WO 2021/054209 (25.03.2021 Gazette 2021/12)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **19.09.2019 JP 2019170710**

(71) Applicant: **Sanyo Chemical Industries, Ltd.
Kyoto-shi, Kyoto 605-0995 (JP)**

(72) Inventors:
• **UEDA, Masumi**
**Kyoto-shi, Kyoto 605-0995 (JP)**
• **SAKUMA, Kohei**
**Kyoto-shi, Kyoto 605-0995 (JP)**
• **TANAKA, Yuya**
**Kyoto-shi, Kyoto 605-0995 (JP)**

(74) Representative: **Mewburn Ellis LLP
Aurora Building
Counterslip
Bristol BS1 6BX (GB)**

(54) **MAGNETIC PARTICLE COMPOSITION, USE OF MAGNETIC PARTICLE COMPOSITION FOR NUCLEIC ACID SEPARATION, KIT FOR OBTAINING MAGNETIC PARTICLE COMPOSITION, MAGNETIC PARTICLES, CHAOTROPIC SALT, AND SEPARATION AND PURIFICATION METHOD**

(57) A magnetic particle composition (e) containing magnetic particles (c) and a chaotropic salt (D), the magnetic particles (c) each including a core particle (P) that is a magnetic silica particle containing a magnetic metal oxide particle (A), wherein the magnetic metal oxide particle (A) in the core particle (P) has a weight percentage of 60 wt% or more based on the weight of the core particle (P), and the magnetic particles (c) have a particle size distribution with a coefficient of variation of 5 to 50%.

EP 4 032 854 A1

(52) Cooperative Patent Classification (CPC): (Cont.)
C01P 2004/84; C01P 2006/42

**Description**

TECHNICAL FIELD

The present invention relates to a magnetic particle composition, use of a magnetic particle composition in nucleic acid separation, a kit for obtaining a magnetic particle composition, magnetic particles, a chaotropic salt, and a separation and purification method.

BACKGROUND ART

Conventionally, spin column purification has been commonly employed as a method of separating genes and the like derived from organisms living in all kinds of environments including water, soil, and air from samples. However, the purification method by spin chromatography involves complicated steps. For example, a substance to be separated needs to be adsorbed onto a solid phase column under centrifugation, and the substance needs to be extracted from the solid phase column. Thus, the spin chromatography is not suitable for quick and simple separation and purification in, for example, agricultural farms, livestock farms, and airports, and real-time analysis has been yet to be achieved. Thus, there is no other choice but to rely on analysis centers for complicated separation and purification using large equipment. This causes a time lag of several days before analysis results are made available. This may worsen the situation on site. For example, the time lag may result in proliferation of harmful bacteria in agricultural farms.

Thus, a technique for purifying a nucleic acid using magnetic silica particles has been disclosed as a technique that allows easy separation and collection by a magnetic force (Non-Patent Literature 1). However, a nucleic acid obtained by this method contains a large amount of impurities other than the target nucleic acid, and the generation efficiency is insufficient.

CITATION LIST

Non-Patent Literature

Non-Patent Literature 1: Procedia Environmental Sciences, 2013, Vol. 18, pp. 856-863

SUMMARY OF INVENTION

- Technical Problem

The present invention aims to provide a magnetic particle composition that can be used in a method of separating a substance to be separated and that enables more efficient obtainment of a high purity product.

- Solution to Problem

As a result of extensive studies to solve the above issues, the present inventors arrived at the present invention.
Specifically, the present invention relates to the followings: a magnetic particle composition (e) containing magnetic particles (c) and a chaotropic salt (D), the magnetic particles (c) each including a core particle (P) that is a magnetic silica particle containing a magnetic metal oxide particle (A), wherein the magnetic metal oxide particle (A) in the core particle (P) has a weight percentage of 60 wt% or more based on the weight of the core particle (P), and the magnetic particles (c) have a particle size distribution with a coefficient of variation of 5 to 50%; use of the magnetic particle composition (e) in nucleic acid separation from soil, environmental water, plant, or animal excrement; a kit (K) for obtaining the magnetic particle composition (e) including a combination of magnetic particles (c) and a chaotropic salt (D), the magnetic particles (c) each including a core particle (P) that is a magnetic silica particle containing a magnetic metal oxide particle (A), wherein the magnetic metal oxide particle (A) in the core particle (P) has a weight percentage of 60 wt% or more based on the weight of the core particle (P), the magnetic particles (c) have a particle size distribution with a coefficient of variation of 5 to 50%, and the magnetic particle composition (e) can be obtained by mixing the magnetic particles (c) and the chaotropic salt (D); magnetic particles (c) for obtaining the magnetic particle composition (e) or the kit (K), wherein the magnetic particles (c) each include a core particle (P) that is a magnetic silica particle containing a magnetic metal oxide particle (A), the magnetic metal oxide particle (A) in the core particle (P) has a weight percentage of 60 wt% or more based on the weight of the core particle (P), and the magnetic particles (c) have a particle size distribution with a coefficient of variation of 5 to 50%; a chaotropic salt (D) for obtaining the magnetic particle composition (e) or the kit (K); and a separation and purification method of separating a substance to be separated (G) from a sample (F) using the magnetic particle composition (e).

- Advantageous Effects of Invention

Use of the magnetic particle composition of the present invention to separate a sample containing a substance to be separated enables efficient obtainment of a high purity product from the sample.

DESCRIPTION OF EMBODIMENTS

(Magnetic particle composition)

The magnetic particle composition of the present invention is a magnetic particle composition (e) containing magnetic particles (c) and a chaotropic salt (D), the magnetic particles (c) each including a core particle (P) that is a magnetic silica particle containing magnetic a metal oxide particle (A), wherein the magnetic metal oxide particle (A) in the core particle (P) has a weight percentage of 60 wt% or more based on the weight of the core particle (P), and the magnetic particles (c) have a particle size distribution with a coefficient of variation of 5 to 50%.

The magnetic particles (c) are suitable for use in a separation method of the present invention (a substance separation method of separating a substance to be separated (G) from a sample (F)) (described later in detail). The substance to be separated (G) in the present invention refers to a target substance (G1) or a non-target substance (G2) in a mixture of multiple substances (e.g., biological substances) in the sample (F).

The magnetic particles (c) are also suitable for a use of a magnetic particle composition (e) of the present invention (described later in detail) in nucleic acid separation.

The target substance (G1) refers to a substance intended to be ultimately obtained as a purified product from the sample (F).

The non-target substance (G2) refers to a substance intended to be ultimately removed from the sample (F).

Examples of the sample (F) herein include samples derived from organisms in the environment (e.g., soil, seawater, plant, or animal excrement, biological fluids (e.g., serum, blood, lymph, ascites, and urine), various cells, and culture solutions) and mixtures containing the target substance (G1) and/or the non-target substance (G2) (described later in detail).

The sample (F) may be soil, environmental water, plant, or animal excrement.

Environmental water is a concept that encompasses water that makes up rivers, lakes, wetlands, sea areas, groundwater, and the like.

The sample (F) may contain a microorganism.

The magnetic particles (c) in the present invention each include the core particle (P) that is a magnetic silica particle. The core particle (P) is a particle containing the magnetic oxide particle (A). The core particle (P) may contain other component(s) as long as it contains the magnetic oxide particle (A).

The magnetic particles (c) in the present invention may be particles each consisting of the core particle (P) that is a magnetic silica particle containing the magnetic metal oxide particle (A).

The core particle (P) herein is the same as the core layer (P) disclosed in Japanese Patent Application No. 2019-170710, which is the priority application of the present application.

The magnetic metal oxide particles (A) in the present invention may be ferrimagnetism, ferromagnetism, or superparamagnetism. Preferred of these is superparamagnetism because there is no residual magnetization derived from magnetic particles with superparamagnetism after magnetic separation so that magnetic particles with superparamagnetism can be quickly re-dispersed. The term "superparamagnetism" is a phenomenon in which a substance induces a temporary magnetic field where individual atomic magnetic moments of the substance are aligned in the presence of an external magnetic field, and the substance loses its magnetic field due to partial misalignment that occurs in response to removal of the external magnetic field.

Examples of the magnetic metal oxide particles (A) include oxides of iron, cobalt, nickel, and alloys thereof. Iron oxides are particularly preferred because they have excellent sensitivity to a magnetic field. These types of the magnetic metal oxide particles (A) may be used alone or in combination of two or more thereof.

The iron oxide used in the magnetic metal oxide particles (A) may be selected from various known iron oxides. Preferred iron oxides are magnetite, $\gamma$-hematite, an intermediate iron oxide between magnetite and $\alpha$-hematite, and an intermediate iron oxide between $\gamma$-hematite and $\alpha$-hematite because they have especially excellent chemical stability. Magnetite is more preferred because it has a high saturation magnetization and excellent sensitivity to an external magnetic field.

The magnetic metal oxide particle (A) has a volume average particle size of preferably 1 to 50 nm, more preferably 1 to 30 nm, particularly preferably 1 to 20 nm. The magnetic metal oxide particle (A) having a volume average particle size of 1 nm or more is easy to synthesize.

Herein, the volume average particle size of the magnetic metal oxide particle (A) is defined as the volume average of particle sizes of any 200 magnetic metal oxide particles (A), as measured by observation using a scanning electron microscope (e.g., "JSM-7000F" from JEOL Ltd.). The volume average particle size of the magnetic metal oxide particle (A) can be controlled by adjusting the metal ion concentration during production of the magnetic metal oxide particles (A) (described later). The volume average particle size of the magnetic metal oxide particle (A) can also be set to a desired value by a method such as classification.

In the magnetic particle composition of the present invention, the magnetic metal oxide particle (A) in the core particle (P) has a weight percentage of 60 wt% or more based on the weight of the core particle (P).

The lower limit of the weight percentage of the magnetic metal oxide particle (A) based on the weight of the core particle (P) is 60 wt%, preferably 65 wt%, and the upper limited thereof is preferably 95 wt%, more preferably 80 wt%.

When the weight percentage of the magnetic metal oxide particle (A) is less than 60 wt%, the resulting magnetic particles (c) have insufficient magnetism, thus requiring a longer separation operation in practical use. When the weight percentage of the magnetic metal oxide particle (A) is more than 95 wt%, synthesis thereof may be difficult.

The magnetic metal oxide particles (A) may be produced by any method. For example, they can be synthesized by co-precipitation using water-soluble iron salts and ammonia based on the method of Massart (R. Massart, IEEE Trans. Magn. 1981, 17, 1247), or by a method utilizing oxidation in an aqueous solution of water-soluble iron salts.

The volume average particle size of the magnetic particles (c) is preferably 0.5 to 20 $\mu$m, more preferably 1 to 10 $\mu$m, particularly preferably 1.1 to 5 $\mu$m.

The magnetic particles (c) having a volume average particle size of 0.5 $\mu$m or more tend to be able to reduce the time for separation and collection. The magnetic particles (c) having an average particle size of 20 $\mu$m or less can each have a relatively large specific surface area, resulting in a larger amount of the substance (G) separated, with a tendency of increased binding efficiency.

Further, the separability of the substance to be separated (G) improves when the magnetic particles (c) have a volume average particle size of 1.1 $\mu$m or more and a particle size distribution with a coefficient of variation of 21 to 35% (described later in detail).

The term "separability" as used herein is described separately for the case where the substance to be separated (G) is the target substance (G1) and for the case where the substance to be separated (G) is the non-target substance (G2) .

When the substance to be separated (G) is the target substance (G1), the expression "separability improves" means that the target substance (G1) in a component extracted from the sample (F) using the magnetic particles (c) has a higher purity (proportion).

When the substance to be separated (G) is the non-target substance (G2), the expression "separability improves" means that the proportion of the non-target substance (G2) in a remaining component is lower after the non-target substance (G2) is removed from the sample (F) using the magnetic particles (c).

In the present invention, the volume average particle size of the magnetic particles (c) is the volume average particle size measured using, for example, a laser diffraction/scattering particle size distribution measuring device ("Microtrac MT3300" from MicrotracBEL Corp.).

The volume average particle size of the magnetic particles (c) can be set to a desired value by changing conditions in a water-washing step or by a method such as classification during production, for example.

In the magnetic particle composition of the present invention, the magnetic particles (c) have a particle size distribution with a coefficient of variation of 5 to 50%.

A coefficient of variation of more than 50% results in poor separability of the substance to be separated (G).

In order to further improve the separability of the substance to be separated (G), the lower limit of the coefficient of variation of the particle size distribution of the magnetic particles (c) is 5% or more, preferably 13% or more, more preferably 20% or more, particularly preferably 21% or more.

In order to further improve the separability of the substance to be separated (G), the upper limit of the coefficient of variation of the particle size distribution of the magnetic particles (c) is preferably 35% or less.

The coefficient of variation of the particle size distribution of the magnetic particles (c) can be measured by the following measurement method.

<Method of measuring coefficient of variation>

The coefficient of variation in the present invention is a value that can be obtained by substituting a volume average particle size (d) and a standard deviation (SD), which are determined by a device such as a laser diffraction/scattering particle size distribution measuring device ("Microtrac MT3300" from MicrotracBEL Corp.), into a formula (1):

$$\text{Coefficient of variation (\%)} = SD/d \times 100 \quad (1)$$

where the volume average particle size (d) is the size of nanometer to micrometer order.

The coefficient of variation of the particle size distribution of the magnetic particles (c) can be adjusted by classifying the magnetic particles (c).

For example, the magnetic particles (c) having a relatively large particle size can be removed by precipitating the particles by centrifugation. The magnetic particles (c) having a relatively small particle size can be removed by removing the supernatant containing non-precipitated fine particles after centrifugation.

The magnetic particle composition of the present invention contains the magnetic particles (c) containing the magnetic metal oxide particles (A) and the chaotropic salt (D). A highly purified product can be efficiently obtained owing to the presence of the magnetic particles (c) and the chaotropic salt (D).

The magnetic particles (c) allow the substance to be separated (G) to be bonded to their surfaces. For example, in a complex of the magnetic particles (c) and a nucleotide chain of DNA that is the substance to be separated (G), the magnetic particles (c) and the nucleotide chain can be bonded via a chaotropic salt (D) (e.g., guanidinium thiocyanate, guanidine hydrochloride, or sodium perchlorate).

The chaotropic salt (D) in the present invention is a substance used for reducing interactions between water molecules present between a surface of each magnetic particle and a nucleic acid substance such as DNA and destabilizing the bonding state. Specifically, it is a solute that increases the entropy of water. Use of the chaotropic salt (D) enables withdrawal of water molecules from the hydrated state of the surface of the magnetic particle and the nucleic acid substance and enables adsorption of DNA onto the surface of the magnetic particle.

In order to increase the purity of the nucleic acid substance, the weight ratio (c/D) of the magnetic particles (c) to the chaotropic salt (D) is preferably 2/98 to 16/84, more preferably 2/98 to 10/90.

The chaotropic salt (D) can be selected from the Hofmeister series. The Hofmeister series is a classification of ions in order of their ability to salt out or salt in proteins, and is also referred to as the lyotropic series. Examples of chaotropic anions include $NO_3^-$, $ClO_4^-$, $SCN^-$, and $NCS^-$. Examples of strong chaotropic cations include $Na^+$, $Ba^{2+}$, and guanidine cation. A preferred chaotropic salt (D) is a salt formed by a combination of one of the above anions and one of the above cations. Examples include guanidinium thiocyanate, guanidine isothiocyanate, guanidinium thiocyanate salt, guanidine hydrochloride, sodium perchlorate, guanidinium thiocyanic acid, and sodium iodide. Of these, use of guanidinium thiocyanate is preferred in terms of yield of the nucleic acid substance.

Here, when the substance to be separated (G) does not directly bind to the magnetic particles (c), a substance (J) that binds to the substance to be separated (G) may be immobilized on surfaces of the magnetic particles (c). The immobilization of the substance (J) on the surfaces allows binding of the substance to be separated (G) to the magnetic particles (c) via the substance (J). Hereinafter, magnetic particles having surfaces with the substance (J) immobilized thereon are also referred to as "magnetic particles (c1)".

The substance to be separated (G) may be the target substance (G1) or the non-target substance (G2). The substance (J) may be any substance that binds to the target substance (G1) or the non-target substance (G2).

The binding between the substance (J) and the substance to be separated (G) may be specific or nonspecific, but preferably, the binding between the substance to be separated (G) and the substance (J) is specific.

In the case where the binding between the substance to be separated (G) and the substance (J) is specific, the separation and purification method using the magnetic particles (c) of the present invention improves the separability of the substance to be separated (G).

Examples of the substance (J) that specifically binds to the substance to be separated (G) include one that binds to the substance to be separated (G) by an interaction such as a reaction between genes.

In the interaction of these substances, when one is the substance to be separated (G), the other is the substance (J) that specifically binds to the substance to be separated (G).

For example, when the substance to be separated (G) is a "gene", the substance (J) is a "gene".

Preferably, the magnetic particle composition of the present invention is for use in nucleic acid separation from soil, environmental water, plant, or animal excrement. Use of the magnetic particle composition of the present invention for separating a nucleic acid from soil, environmental water, plant, or animal excrement has been unknown. No suggestions have been made on suitability of the magnetic particle composition of the present invention for such use.

Use of the magnetic particle composition in nucleic acid separation from soil, environmental water, plant, or animal excrement is encompassed by the present invention.

Preferably, in the magnetic particle composition of the present invention, the magnetic particles (c) are core-shell particles (C) each including the core particle (P) that is a magnetic silica particle containing the magnetic metal oxide particle (A) and a shell layer (Q) that is a silica layer having an average thickness of 3 to 3000 nm formed on a surface of the core particle (P), and the magnetic particle composition is a mixture (E) of the core-shell particles (C) and the chaotropic salt (D).

The following describes an example of an embodiment of the magnetic particle composition of the present invention, in which the magnetic particles (c) are the core-shell particles (C), and the magnetic particle composition (e) is the mixture (E) of the core-shell particles (C) and the chaotropic salt (D).

The mixture (E) is a mixture of the core-shell particles (C) and the chaotropic salt (D).

The core-shell particles (C) are core-shell particles each including the core particle (P) that is a magnetic silica particle containing the magnetic metal oxide particle (A) and the shell layer (Q) that is a silica layer having an average thickness of 3 to 3000 nm formed on the surface of the core particle (P).

The magnetic metal oxide particle (A) in the core particle (P) has a weight percentage of 60 to 95 wt% based on the weight of the core particle (P). The core-shell particles (C) have a particle size distribution with a coefficient of variation of 5 to 50%.

The core-shell particles (C) are particularly suitable for use in the separation and purification method of the present invention (a substance separation method of separating a substance to be separated (G) from a sample (F)) (described later in detail). The substance to be separated (G) in the present invention refers to the target substance (G1) or the non-target substance (G2) in a mixture of multiple substances (e.g., biological substances) in the sample (F).

The target substance (G1) refers to a substance intended to be ultimately obtained as a purified product from the sample (F).

The non-target substance (G2) refers to a substance intended to be ultimately removed from the sample (F).

Examples of the sample (F) include samples derived from organisms in the environment (e.g., soil, seawater, plant, or animal excrement, biological fluids (e.g., serum, blood, lymph, ascites, and urine), various cells, and culture solutions) and mixtures containing the target substance (G1) and/or the non-target substance (G2) (described later in detail).

The sample (F) may be soil, environmental water, plant, or animal excrement. The sample (F) may contain a microorganism.

As described above, the core-shell particles (C) each include the core particle (P) that is a magnetic silica particle containing the magnetic metal oxide particle (A) and the shell layer (Q) that is a silica layer having an average thickness of 3 to 3000 nm formed on the surface of the core particle (P). Preferably, the core particle (P) is a sphere containing the magnetic metal oxide particle (A) dispersed in a silica matrix. The shell layer (Q) may contain a component in addition to silica.

The magnetic metal oxide particle (A) in the core particle (P) preferably has a volume average particle size of 1 to 50 nm, more preferably 1 to 30 nm, particularly preferably 1 to 20 nm. The magnetic metal oxide particles (A) having a volume average particle size of 1 nm or more are easy to synthesize. The magnetic metal oxide particles (A) having a volume average particle size of 50 nm or less are easily dispersed uniformly in a silica matrix.

In the present invention, the lower limit of the weight percentage of the magnetic metal oxide particle (A) based on the weight of the core particle (P) is 60 wt%, preferably 65 wt%, and the upper limit thereof is 95 wt%, preferably 80 wt%.

When the weight percentage of the magnetic metal oxide particle (A) is less than 60 wt%, the resulting core-shell particles (C) have insufficient magnetism, thus requiring a longer separation operation in practical use. When the weight percentage of the magnetic metal oxide particle (A) is more than 95 wt%, synthesis thereof is difficult.

As described above, the core-shell particles (C) are core-shell particles in which the shell layer (Q) is formed on the surface of each core particle (P).

The average thickness of the shell layers (Q) can be measured by transmission electron microscopic observation of microtome cross sections of the core-shell particles (C) embedded in a resin (e.g., epoxy resin) and analyzing images obtained by the observation. The average thickness of the shell layers (Q) is defined as the average of the thicknesses of the shell layers (Q) of any 100 core-shell particles (C), as measured by observation using a transmission electron microscope (e.g., "H-7100" from Hitachi, Ltd.). The thickness of the shell layer (Q) is an average of the thinnest and thickest portions thereof in one core-shell particle (C). The average thickness of the shell layers (Q) of each of 100 particles is determined by a similar method, and further, the average of 100 particles is calculated, whereby the thickness of the shell layer (Q) is determined.

The average thickness of the shell layers (Q) is 3 to 3000 nm, preferably 10 to 800 nm, more preferably 50 to 800 nm, particularly preferably 50 to 500 nm, most preferably 50 to 200 nm. When the average thickness of the shell layers (Q) is less than 3 nm, no effect is achieved by the formation of the shell layers (Q), resulting in a smaller amount of the substance (G) separated. The shell layers (Q) having an average thickness of more than 3000 nm are difficult to synthesize.

In some cases, the shell layer (Q) and the core particle (P) are difficult to distinguish therebetween in analysis of the images obtained by observation under the transmission electron microscope.

The particles of the present invention, which contain magnetic metal oxide particles, can be used for separation and purification because they form a composite with a target substance or a non-target substance. Each particle is only required to have a suitable amount of silanol groups on its surface, and preferably, the amount of metal atoms derived from the magnetic metal oxide particle (A) is 10 mol% or less relative to the total atoms on the surface of the particle. The ratio (mol%) can be measured by X-ray photoelectron spectroscopy (XPS).

The volume average particle size of the core-shell particles (C) is preferably 0.5 to 20 $\mu$m, more preferably 1 to 10 $\mu$m, particularly preferably 1.1 to 5 $\mu$m.

The core-shell particles (C) having a volume average particle size of 0.5 $\mu$m or more tend to reduce the time for separation and collection. The core-shell particles (C) having an average particle size of 20 µm or less can each have a relatively large specific surface area, resulting in a larger amount of the substance (G) separated, with a tendency of increased binding efficiency.

Further, the separability of the substance to be separated (G) improves when the core-shell particles (C) have a volume average particle size of 1.1 µm or more and a particle size distribution with a coefficient of variation of 21 to 35% (described in detail later).

The volume average particle size of the core-shell particles (C) is the volume average particle size measured using, for example, a laser diffraction/scattering particle size distribution measuring device ("Microtrac MT3300" from MicrotracBEL Corp.).

The volume average particle size of the core-shell particles (C) can be controlled by controlling the volume average particle size of the core particles (P) and the average thickness of the shell layers (Q). The volume average particle size of the core particles (P) can be controlled by adjusting mixing conditions (e.g., shear strength) in production of an oil-in-water emulsion (described later) to adjust the particle size of the oil-in-water emulsion. The average thickness of the shell layers (Q) can be controlled by adjusting the amount of an (alkyl)alkoxysilane, the amount of a catalyst, reaction time, and the like during formation of the shell layers (Q) (described later).

The volume average particle size of the core particles (P) and the volume average particle size of the core-shell particles (C) can also be set to desired values by changing conditions in a water-washing step or by a method such as classification during production, for example.

In the present invention, the core-shell particles (C) have a particle size distribution with a coefficient of variation of 50% or less, as described above. A coefficient of variation of more than 50% results in poor separability of the substance to be separated (G).

In order to further improve the separability of the substance to be separated (G), the lower limit of the coefficient of variation of the particle size distribution of the core-shell particles (C) is preferably 5% or more, more preferably 13% or more, still more preferably 20% or more, particularly preferably 21% or more.

In order to further improve the separability of the substance to be separated (G), the upper limit of the coefficient of variation of the particle size distribution of the core-shell particles (C) is 35% or less.

The coefficient of variation of the particle size distribution of the core-shell particles (C) can be measured by the following measurement method.

<Method of measuring coefficient of variation>

The coefficient of variation in the present invention is a value that can be obtained by substituting a volume average particle size (d) and a standard deviation (SD), which are determined by a device such as a laser diffraction/scattering particle size distribution measuring device ("Microtrac MT3300" from MicrotracBEL Corp.), into a formula (1):

$$\text{Coefficient of variation (\%)} = SD/d \times 100 \quad (1)$$

where the volume average particle size (d) is the size of nanometer to micrometer order.

The coefficient of variation of the particle size distribution of the core-shell particles (C) can be adjusted by classifying the core-shell particles.

For example, core-shell particles having a relatively large particle size can be removed by precipitating the particles by centrifugation. Core-shell particles having a relatively small particle size can be removed by removing the supernatant containing non-precipitated fine particles after centrifugation.

Preferably, the core-shell particles (C) of the present invention each have a ratio of the average thickness of the shell layers (Q) to the particle size of the core particle (P) (average thickness of shell layers (Q)/particle size of core particle (P)) of 0.001 to 10, more preferably 0.02 to 1.5, particularly preferably 0.04 to 1.5.

The separability of the substance to be separated (G) improves when the above ratio is 0.001 or more. The separability of the substance to be separated (G) improves when the ratio is 10 or less. Here, in calculation of the ratio, the average thickness of the shell layers (Q) is a value determined by the method described above. In calculation of the ratio, the particle size of the core particle (P) can be determined by the following calculation formula, using values of "the volume average particle size of the core-shell particles (C)" and "the average thickness of the shell layers (Q)" described above.

Particle size of core particle (P) = (Volume average particle size of core-shell particles (C)) - 2 × (Average thickness of shell layers (Q))

The separability of the substance to be separated (G) improves significantly when the core-shell particles (C) have a ratio of the average thickness of the shell layers (Q) to the particle size of the core particle (P) (average thickness of shell layers (Q)/particle size of core particle (P)) of 0.02 to 1.5 and a particle size distribution with a coefficient of variation of 21 to 35%.

The core-shell particles (C) in the present invention each include the shell layer (Q) that is a silica layer, so that each core-shell particle (C) has a silanol group on its surface. Thus, the substance to be separated (G) of a predetermined type can be bonded to the surface of the particle.

As described above, the core-shell particles (C) allow the substance to be separated (G) to be bonded to their surfaces due to the silanol groups of the core-shell particles (C). In one specific method, for example, in a complex of the silanol groups of the core-shell particles (C) and a nucleotide chain of DNA that is the substance to be separated (G), the silanol groups and the nucleotide chain can be bonded via a chaotropic salt (D) (e.g., guanidinium thiocyanate, guanidine hydrochloride, or sodium perchlorate).

In the case where the substance to be separated (G) does not directly bind to the core-shell particles (C), the substance (J) that binds to the substance to be separated (G) may be immobilized on surfaces of the core-shell particles (C). The immobilization of the substance (J) on the surfaces allows binding of the substance to be separated (G) to the core-shell particles (C) via the substance (J). Hereinafter, core-shell particles having surfaces with the substance (J) immobilized thereon are also referred to as "core-shell particles (C1)".

The substance to be separated (G) may be the target substance (G1) or the non-target substance (G2). The substance (J) may be any substance that binds to the target substance (G1) or the non-target substance (G2).

The binding between the substance (J) and the substance to be separated (G) may be specific or nonspecific, but preferably, the binding between the substance (J) and the substance to be separated (G) is specific.

In the case where the binding between the substance to be separated (G) and the substance (J) is specific, the separation and purification method using the core-shell particles (C) of the present invention improves the separability of the substance to be separated (G).

Examples of the substance (J) that specifically binds to the substance to be separated (G) include one that binds to the substance to be separated (G) by an interaction such as a reaction between genes.

In the interaction of these substances, when one is the substance to be separated (G), the other is the substance (J) that specifically binds to the substance to be separated (G).

For example, when the substance to be separated (G) is a "gene", the substance (J) is a "gene".

Next, the method of producing the magnetic particles (c) of the present invention is described with reference to a method of producing the core-shell particles (C) as the magnetic particles (c) as a typical example. The core-shell particles (C) can be produced by a production method involving at least two steps described below.

(Step 1) A step of producing an oil-in-water emulsion of an (alkyl)alkoxysilane containing the magnetic metal oxide particles (A) to cause hydrolysis and polycondensation reaction of the (alkyl)alkoxysilane to produce the core particles (P) containing the magnetic metal oxide particles (A) embedded in silica.

(Step 2) A step of causing hydrolysis and polycondensation reaction of an (alkyl)alkoxysilane on the surface of each core particle (P) to form the shell layer (Q) thereon

The above steps are described below.

First, step 1 is described.

Examples of the production method of the core particles (P) include a method in which an oil-in-water emulsion is produced by mixing a dispersion (B1) containing the magnetic metal oxide particles (A) and an (alkyl)alkoxysilane in an amount of 30 to 1000 wt% based on the weight of the magnetic metal oxide particles (A) (hereinafter, such a dispersion is also simply referred to as the "dispersion (B1)") with a solution (B2) containing water, a nonionic surfactant, and a catalyst for hydrolysis of the (alkyl)alkoxysilane (hereinafter, such a solution is also simply referred to as the "the solution (B2)") to cause hydrolysis and polycondensation reaction of the (alkyl)alkoxysilane so as to produce particles containing the magnetic metal oxide particles (A) embedded in silica.

After the hydrolysis and polycondensation reaction of the (alkyl)alkoxysilane, the resulting product is subjected to solid-liquid separation by centrifugation and using a magnet or the like, whereby the core particles (P) are obtained.

In the above and below, the (alkyl)alkoxysilane refers to alkylalkoxysilane and/or alkoxysilane.

Examples of the (alkyl)alkoxysilane used include a compound represented by the following formula (1):

$$R_{1(4-n)}Si(OR_2)n \qquad (1)$$

In the formula (1), $R_1$ and $R_2$ each represent a C1-C10 monovalent hydrocarbon group. One or more hydrogen atoms of the hydrocarbon group may be replaced by amino, carboxy, hydroxy, mercapto, or glycidyloxy groups.

Examples of the C1-C10 monovalent hydrocarbon group include C1-C10 aliphatic hydrocarbon groups (e.g., methyl, ethyl, n- or iso-propyl, n- or iso-butyl, n- or iso-pentyl, and vinyl groups), C6-C10 aromatic hydrocarbon groups (e.g., phenyl group), and C7-C10 aromatic-aliphatic groups (e.g., benzyl group).

In formula (1), n is an integer of 1 to 4. Use of an alkylalkoxysilane in which n is 1 requires use of an (alkyl)alkoxysilane in which n is 2 to 4 in combination. Preferably, n is 4 in terms of particle strength and amount of silanol groups on particle surfaces after the reaction.

Specific examples of the compound represented by formula (1) include alkoxysilanes (e.g., tetramethoxysilane, tetraethoxysilane, tetraisopropoxysilane, and tetrabutoxysilane); alkylalkoxysilanes (e.g., methyltrimethoxysilane and methyltriethoxysilane); alkylalkoxysilanes having an amino-substituted alkyl group (e.g., 3-aminopropyltrimethoxysilane, 3-aminopropylethoxysilane, N-(2-aminoethyl)-3-aminopropyltrimethoxysilane, and N-(2-aminoethyl)-3-aminopropyltriethoxysilane); alkylalkoxysilanes having a carboxy-substituted alkyl group (e.g., 7-carboxy-heptyltriethoxysilane and 5-carboxy-pentyltriethoxysilane); alkylalkoxysilanes having a hydroxy-substituted alkyl group (e.g., 3-hydroxypropyltrimethoxysilane and 3-hydroxypropyltriethoxysilane); alkylalkoxysilanes having a mercapto-substituted alkyl group (e.g., 3-mercaptopropyltrimethoxysilane and 3-mercaptopropyltriethoxysilane); alkylalkoxysilanes having a glycidyloxy-subsituted alkyl group (e.g., 3-glycidyloxypropyltrimethoxysilane and 3-glycidyloxypropyltriethoxysilane).

These (alkyl)alkoxysilanes may be used alone or in combination of two or more thereof.

The amount of the (alkyl)alkoxysilane is preferably 30 to 1000 wt%, more preferably 40 to 500 wt%, relative to the weight of the magnetic metal oxide particles (A). Use of the (alkyl)alkoxysilane in an amount of 30 wt% or more relative to the weight of the magnetic metal oxide particles (A) facilitates uniform coating on surfaces of the magnetic metal oxide particles (A). Use of the (alkyl)alkoxysilane in an amount of 1000 wt% or less relative to the weight of the magnetic metal oxide particles (A) can reduce the time for collection by a magnetic force.

The amount of water is preferably 500 to 50000 wt%, more preferably 1000 to 10000 wt%, relative to the weight of the magnetic metal oxide particles (A).

Further, synthesis of the core particles (P) may include adding a water-soluble organic solvent or the like to the solution (B2) or the like.

Examples of the water-soluble organic solvent include those having a solubility in water at 25°C of at least 100 g/100 g of water, such as C1-C4 monohydric alcohols (e.g., methanol, ethanol, and n- or iso-propanol), C2-C9 glycols (e.g., ethylene glycol and diethylene glycol), amides (e.g., N-methylpyrrolidone), ketones (e.g., acetone), cyclic ethers (e.g., tetrahydrofuran and tetrahydropyran), lactones (e.g., γ-butyrolactone), sulfoxides (e.g., dimethylsulfoxide), and nitriles (e.g., acetonitrile).

Preferred of these are C1-C4 monohydric alcohols for a uniform particle size of the core-shell particles (C). These water-soluble organic solvents may be used alone or in combination of two or more thereof.

The amount of the water-soluble organic solvent is preferably 100 to 500 wt% relative to the weight of the water.

Examples of the nonionic surfactant include: adducts of alkylene oxides (hereinafter, an alkylene oxide is abbreviated to "AO") with C8-C24 monohydric alcohols (e.g., decyl alcohol, dodecyl alcohol, coconut oil alkyl alcohol, octadecyl alcohol, and oleyl alcohol); adducts of AO with C3-C36 dihydric to octahydric alcohols (e.g., glycerol, trimethylolpropane, pentaerythritol, sorbitol, and sorbitan); adducts of AO with alkylphenols having a C6-C24 alkyl group (e.g., octylphenol and nonylphenol); adducts of ethylene oxide with polypropylene glycol, and adducts of propylene oxide with polyethylene glycol; adducts of AO with C8-C24 fatty acids (e.g., decanoic acid, lauric acid, myristic acid, palmitic acid, stearic acid, oleic acid, and coconut oil fatty acid); fatty acid esters of the C3-C36 dihydric to octahydric alcohols and AO adducts thereof (e.g., TWEEN® 20 and TWEEN® 80); alkyl glucosides (e.g., N-octyl-β-D-maltoside, n-dodecanoylsucrose, and n-octyl-β-D-glucopyranoside); and fatty acid esters of sucrose, fatty acid alkanolamides, and AO adducts thereof (e.g., polyoxyethylene fatty acid alkanolamides).

These may be used alone or in combination of two or more thereof. Examples of the AO in the description of the nonionic surfactant include ethylene oxide, propylene oxide, and butylene oxide. The AO may be added in a block or random form. The number of moles of AO added is preferably 1 to 50, more preferably 1 to 20, per mole of alcohol, phenol, or fatty acid.

In terms of solubility in water and viscosity, preferred of these nonionic surfactants are adducts of 1 to 50 mol (preferably 1 to 20 mol) ethylene oxide with a C8-C24 monohydric alcohol, such as polyoxyethylene alkyl ether and polyoxyethylene alkyl ether.

In order to improve the separability of the substance to be separated (G) upon separation and purification using the ultimately obtained core-shell particles (C), preferred of these nonionic surfactants are adducts of 1 to 50 mol (preferably 1 to 20 mol) ethylene oxide with a monohydric alcohol having a C8-C24 alkenyl group (e.g., oleyl alcohol) .

The amount of the nonionic surfactant is 10 to 1000 wt%, more preferably 100 to 500 wt%, relative to the weight of the magnetic metal oxide particles (A). Use of the nonionic surfactant in an amount of 10 wt% or more or 1000 wt% or less relative to the weight of the magnetic metal oxide particles (A) tends to stabilize the emulsion and narrow the particle size distribution of the resulting particles.

The amount of the solution (B2) used in step 1 is preferably 1000 to 10000 wt%, more preferably 1500 to 4000 wt%, relative to the weight of the magnetic metal oxide particles (A) in the dispersion (B1).

Use of an aqueous solution containing the nonionic surfactant in an amount of 1000 wt% or more or 10000 wt% or less relative to the weight of the magnetic metal oxide particles (A) tends to stabilize the emulsion and narrow the particle size distribution of the resulting particles.

The catalyst for hydrolysis of the (alkyl)alkoxysilane can be a Lewis acid or a hydrochloric acid, for example. Specific examples thereof include inorganic acids (e.g., hydrochloric acid), organic acids (e.g., acetic acid), inorganic base compounds (e.g., ammonia), and amine compounds (e.g., ethanolamine). The amount of the catalyst for hydrolysis is preferably 1 to 1000 wt%, more preferably 2 to 500 wt%, relative to the weight of the (alkyl)alkoxysilane.

The dispersion (B1) and the solution (B2) may be mixed by any method. They can be mixed at once using an apparatus (described later), but it is preferred to add the dispersion (B1) dropwise to the solution (B2) under stirring for a uniform particle size of the core-shell particles (C).

The apparatus for mixing the dispersion (B1) with the solution (B2) may be any commercially available emulsifier or disperser. Examples thereof include batch-type emulsifiers such as IKA Homogenizer (IKA), Polytron (Kinematica), and TK Auto Homomixer (PRIMIX Corporation); continuous-type emulsifiers such as Ebara Milder (Ebara Corporation), TK Fill Mix, TK Pipeline Homomixer (PRIMIX Corporation), a colloid mill (Kobelco Eco-Solutions Co., Ltd.), Clearmix (M Technique Co., Ltd.), Slasher and Trigonal wet-type grinding machines (Nippon Coke & Engineering, Co., Ltd.), Cavitron (Eurotec Co., Ltd.), and Fine Flow Mill (Pacific Machinery & Engineering Co., Ltd.); high-pressure emulsifiers such as Microfluidizer (Mizuho Industrial Co., Ltd.), Nanomizer Inc. (Nanomizer Inc.), and APV Gaulin (Gaulin); membrane emulsifiers such as a membrane emulsifier (REICA Co., Ltd.); vibration-type emulsifiers such as Vibromixer (REICA Co., Ltd.); and ultrasonic emulsifiers such as an ultrasonic homogenizer (Branson).

Preferred of these are APV Gaulin, IKA homogenizer, TK Auto Homomixer, Ebara Milder, TK Fill Mix, TK Pipeline Homomixer, and Clearmix (M Technique) for a uniform particle size.

The temperature of hydrolysis and polycondensation reaction of the (alkyl)alkoxysilane is preferably 10°C to 100°C, more preferably 25°C to 60°C. The reaction time is preferably 0.5 to 5 hours, more preferably 1 to 2 hours.

Next, step 2 is described. Examples of the method of forming the shell layers (Q) include a method in which the core particles (P) obtained in step 1, an

(alkyl)alkoxysilane, a catalyst for hydrolysis of the (alkyl)alkoxysilane, water, and if necessary, a water-soluble organic solvent are mixed to cause hydrolysis and polycondensation reaction of the (alkyl)alkoxysilane, whereby the shell layers (Q) containing silica are formed on the surfaces of the core particles (P).

Examples of the (alkyl)alkoxysilane hydrolysis used in step 2 include the (alkyl)alkoxysilanes exemplified in the description of step 1. Preferred examples are also as described above.

In the reaction to form the shell layers (Q), the concentration of the core particles (P) is preferably less than 50 wt%, more preferably less than 20 wt%, based on the weight of the reaction solution.

The core particles (P) having a concentration of less than 50 wt% are uniformly dispersed in the solution. This facilitates uniform formation of the shell layers (Q), and can prevent or reduce silica-mediated aggregation of the core particles (P).

In the reaction to form the shell layer (Q), the concentration of the (alkyl)alkoxysilane is preferably less than 50 wt%, more preferably less than 20 wt%, based on the weight of the reaction solution.

A concentration of the (alkyl)alkoxysilane of less than 50 wt% in the solution can prevent or reduce silica-meditated aggregation of the core particles (P), and can also prevent or reduce generation of particles consisting of silica, aggregates of such particles, and aggregates consisting of such particles and the core particles (P).

Example of the catalyst for hydrolysis of the (alkyl)alkoxysilane used in step 2 include the catalysts for hydrolysis exemplified in the description of step 1.

The amount of the catalyst for hydrolysis is preferably 1 to 2000 wt%, more preferably 2 to 1000 wt%, relative to the weight of the (alkyl)alkoxysilane.

The amount of water is preferably 0.01 to 99.9 wt%, more preferably 0.1 to 99.9 wt%, relative to the weight of the reaction solution (the total weight of the core particles (P), (alkyl)alkoxysilane, catalyst for hydrolysis, water, and water-soluble organic solvent used in the reaction).

Use of water in an amount of 0.01 wt% or more relative to the weight of the (alkyl)alkoxysilane can reduce the reaction time for forming the shell layers (Q) having a desired average thickness, without excessively slowing down the reaction speed of hydrolysis of the (alkyl)alkoxysilane.

The water-soluble organic solvent may or may not be used. When used, the water-soluble organic solvents may be used alone or in combination of two or more thereof.

Examples of the water-soluble organic solvent include the water-soluble organic solvents exemplified in the description of step 1. Preferred examples are also as described above.

In addition to the above, a nonionic surfactant or the like can also be used to improve the dispersibility of the core particles (P) during reaction.

Examples of the nonionic surfactant include the nonionic surfactants exemplified in the description of step 1. Preferred examples are also as described above.

The temperature of the hydrolysis and polycondensation reaction of the (alkyl)alkoxysilane in step 2 is preferably 0°C to 90°C, more preferably 15°C to 50°C. The reaction time of the hydrolysis and polycondensation reaction of the (alkyl)alkoxysilane in step 2 is preferably 1 to 5 hours, more preferably 1 to 3 hours.

In the case of producing the magnetic particles (c) of the present invention that are not the core-shell particles (C), only step 1 (step of producing the core particle (P)) of the method of producing the core-shell particles (C) described above is performed, and step 2 (step of producing the shell layer (Q)) is not performed. The magnetic particles (c) that are not the core-shell particles (C) can be obtained by performing only step 1.

Next, a kit for obtaining the magnetic particle composition (e) of the present invention is described.

A kit (K) of the present invention is a kit (K) for obtaining a magnetic particle composition (e),

the kit including a combination of magnetic particles (c) and a chaotropic salt (D), the magnetic particles (c) each including a core particle (P) that is a magnetic silica particle containing a magnetic metal oxide particle (A), wherein the magnetic metal oxide particle (A) in the core particle (P) has a weight percentage of 60 wt% or more based on the weight of the core particle (P),

the magnetic particles (c) have a particle size distribution with a coefficient of variation of 5 to 50%, and the magnetic particle composition (e) can be obtained by mixing the magnetic particles (c) and the chaotropic salt (D).

In the kit (K), the magnetic particles (c) and the chaotropic salt (D) for obtaining the magnetic particle composition (e) are present separately.

Immediately before use of the magnetic particle composition (e), the magnetic particles (c) and the chaotropic salt (D) are mixed to obtain the magnetic particle composition (e).

Preferably, the magnetic particles (c) are the core-shell particles (C). Preferably, the kit provides a mixture (E) obtainable by mixing the core-shell particles (C) and the chaotropic salt (D).

The magnetic particles (c) of the present invention are the magnetic particles (c) for obtaining the magnetic particle composition (e) of the present invention or the kit (K) of the present invention,

wherein the magnetic particles (c) each include a core particle (P) that is a magnetic silica particle containing a magnetic metal oxide particle (A),

the magnetic metal oxide particle (A) in the core particle (P) has a weight percentage of 60 wt% or more based on the weight of the core particle (P), and

the magnetic particles (c) have a particle size distribution with a coefficient of variation of 5 to 50%.

Use of the magnetic particles (c) of the present invention enables obtainment of the magnetic particle composition (e) of the present invention or the kit (K) of the present invention.

Preferably, the magnetic particles (c) are the core-shell particles (C).

The chaotropic salt (D) of the present invention is a chaotropic salt (D) for obtaining the magnetic particle composition (e) of the present invention or the kit (K) of the present invention.

Use of the chaotropic salt (D) of the present invention enables obtainment of the magnetic particle composition (e) of the present invention or the kit (K) of the present invention.

Next, the separation and purification method of the present invention is described.

The separation and purification method of the present invention is a separation and purification method of separating the substance to be separated (G) from a sample (F) using the magnetic particle composition (e) of the present invention.

The following describes a separation and purification method of separating the substance to be separated (G) from the sample (F) using the magnetic particles (c).

Preferably, the sample (F) for use in the separation and purification method of the present invention is soil, environmental water, plant, or animal excrement. The separation and purification method of the present invention is suitable for separating a substance to be separated from such samples.

In the method of separating and purifying the substance to be separated (G) of the present invention according to a first embodiment, the substance to be separated (G) is the target substance (G1). The method includes a composite formation step (1) of forming a composite (H1) of the magnetic particles (c) and the target substance (G1) by contacting a sample (F1) containing the target substance (G1) with the magnetic particle composition (e) of the present invention; a composite separation step (2) of separating the composite (H1) from the sample (F1) by a magnetic force; and a target substance dissociation step (3) of obtaining the target substance (G1) from the composite (H1) by adding a dissociation solution (I).

(Separation and purification method)

In the separation and purification method of the present invention according to the first embodiment, the substance to be separated (G) is the target substance (G1), and it is a method of extracting and purifying the target substance (G1) from the sample (F1) containing the target substance (G1).

The separation and purification method of the present invention according to the first embodiment includes the composite formation step (1), the composite separation step (2), and the target substance dissociation step (3).

Each step is described below.

(1) Composite formation step

In this step, the sample (F) containing the target substance (G1) and the non-target substance (G2) is contacted with the magnetic particle composition (e) containing the magnetic particles (c) and the chaotropic salt (D) to form a composite (H1) of the magnetic particles (c) and the target substance (G1). The chaotropic salt (D) may be present in the bonded state as a counter ion to the composite (H1) or may be present in a solution containing the composite (H1). The method of removing the non-target substance (G2) is described later.

In the composite formation step, preferably, the sample (F1) containing the target substance (G1) is contacted with the magnetic particle composition (e) in the presence of ethanol. This promotes adsorption of the target substance (G1) onto the magnetic particles (c).

(2) Composite separation step

Next, the composite (H1) is separated from the sample (F1) by a magnetic force. The composite (H1) contains the magnetic particles (c), and the magnetic particles (c) contain the magnetic metal oxide particles (A). Thus, the composite (H1) can be collected by a magnetic force. Subsequently, the remaining sample (F1) is removed, whereby the composite (H1) can be separated from the sample (F1).

Such a separation method may be, for example, one that includes collecting the composite (H1) by a magnetic force of a magnet or the like from the outside of a reaction vessel, discharging the supernatant, and separating the composite (H1).

(3) Target substance dissociation step

Next, the target substance (G1) is obtained by dissociating the target substance (G1) from the composite (H1) .

The target substance (G1) may be dissociated from the composite (H1) by any method, such as one in which a substance that inhibits binding between the magnetic particles (c) and the target substance (G1) is added to dissociate the target substance (G1). The substance that inhibits binding between the magnetic particles (c) and the target substance (G1) varies depending on the types of the target substance (G1) and the substance (G). Examples thereof include substances that inhibit binding by the pH difference, salinity difference, or action of a surfactant. Examples of the substance capable of inhibiting binding (dissociation solution I) include water, ethanol, IPA, and an aqueous TE-HC1 buffer solution.

When the substance to be separated (G) is the non-target substance (G2), it is possible to use a separation and purification method including: a composite formation step of forming a composite (H2) of the magnetic particles (c) and the non-target substance (G2) by contacting a sample (F2) containing the target substance (G1) and the non-target substance (G2) with the magnetic particle composition (e); and a non-target substance removal step of removing the non-target substance (G2) from the sample (F2) by separating the composite (H2) from the sample (F2) by a magnetic force to obtain a sample (F3) containing the target substance (G1).

The method of separating and purifying the substance to be separated (G) according to a second embodiment of the present invention includes: a composite formation step of forming a composite (H2) of the magnetic particles (c) and the non-target substance (G2) by contacting a sample (F2) containing the target substance (G1) and the non-target substance (G2) with the magnetic particle composition (e) of the present invention; and a non-target substance removal step of removing the non-target substance (G2) from the sample (F2) by separating the composite (H2) from the sample (F2) by a magnetic force to obtain a sample (F3) containing the target substance (G1).

In the separation and purification method of the present invention according to the second embodiment, the substance to be separated (G) is the non-target substance (G2), and it is a method of removing the non-target substance (G2) from the sample (F2) containing the non-target substance (G2).

The separation and purification method of the present invention include (1) the composite formation step and (2) the non-target substance removal step.

Each step is described below.

(1) Composite formation step

In this step, the sample (F) containing the target substance (G1) and the non-target substance (G2) is contacted with the magnetic particle composition (e) containing the magnetic particles (c) and the chaotropic salt (D) to form a composite (H1) of the magnetic particles (c) and the target substance (G1) and a composite (H2) of the magnetic particles (c) and the non-target substance (G2) .

The composite (H2) may be formed by direct binding of the non-target substance (G2) to the magnetic particles (c). Alternatively, the magnetic particles (c) may contain the substance (G) that binds to the non-target substance (G2), and the composite (H2) may be formed by binding of the non-target substance (G2) to the magnetic particles (c) via the substance (G). The chaotropic salt (D) may be present in the bonded state as a counter ion to the composite (H1) and the composite (H2) or may be present in a solution containing the composite (H1) and the composite (H2) .

(2) Non-target substance removal step

Next, the composite (H1) and the composite (H2) are separated from the sample (F2) by a magnetic force. The composite (H1) and the composite (H2) contain the magnetic particles (c), and the magnetic particles (c) contain the magnetic metal oxide particles (A). Thus, the composite (H1) and the composite (H2) can be collected by a magnetic force. In the composite (H2), the non-target substance (G2) exhibits weaker adsorption than the target substance (G1) onto the magnetic particles (c), and the non-target substance (G2) is dissociated from the composite (H2) and moves into a sample solution during magnetic collection. The composite (H1) and the composite (H2) are collected by a magnetic force of a magnet or the like from the outside of a reaction vessel. Then, the sample solution is stirred, and the supernatant is discharged. Thus, the composite (H1) is magnetically collected from the outside of the reaction vessel and remains as the residue in the reaction vessel, while the non-target substance (G2) forming the composite (H2) is contained in the supernatant. The non-target substance (G2) can be separated by this method.
In the separation and purification method of the present invention, examples of the target substance (G1) include single-stranded DNA, double-stranded DNA, single-stranded RNA, and double-stranded RNA.
The non-target substance (G2) in the present invention refers to at least one substance in the sample (F), excluding the target substance (G1). In other words, the non-target substance (G2) may include multiple types of non-target substances (G2).
For example, when the sample (F) is soil and the target substance (G1) is single-stranded DNA derived from a microorganism in the soil, the non-target substance (G2) is at least one of other components (e.g., proteins (e.g., albumin), fats, and inorganic substances) in the soil.
In the present invention, the substance to be separated (G) is preferably a nucleic acid, and is more preferably at least one selected from the group consisting of single-stranded DNA, double-stranded DNA, single-stranded RNA, a double-stranded RNA virus, a bacterium, and a protein.
Also, the substance to be separated (G) is preferably at least one selected from the group consisting of DNA and RNA.
In the separation and purification method of the present invention, preferably, the magnetic particles (c) are the core-shell particles (C), and preferably, the substance to be separated (G) is separated from the sample (F) using the mixture (E) of the core-shell particles (C) and the chaotropic salt (D).
The following describes the separation and purification method of separating the substance to be separated (G) from the sample (F) using the core-shell particles (C).
In this case, the separation and purification method according to the first embodiment includes the following steps when the substance to be separated (G) is the target substance (G1): a composite formation step (1) of forming a composite (H1) of the core-shell particles (C) and the target substance (G1) by contacting a sample (F1) containing the target substance (G1) with the mixture (E); a composite separation step (2) of separating the composite (H1) from the sample (F1) by a magnetic force; and a target substance dissociation step (3) of obtaining the target substance (G1) from the composite (H1) by adding a dissociation solution (I).

(Separation and purification method)

In the separation and purification method, the substance to be separated (G) is the target substance (G1), and it is a method of extracting and purifying the target substance (G1) from a sample (F1) containing the target substance (G1).
The separation and purification method includes (1) a composite formation step, (2) a composite separation step, and (3) a target substance dissociation step.
Each step is described below.

(1) Composite formation step

In this step, the sample (F) containing the target substance (G1) and the non-target substance (G2) is contacted with a mixture (E) of the core-shell particles (C) and the chaotropic salt (D) to form a composite (H1) of the core-shell particles (C) and the target substance (G1). The chaotropic salt (D) may be present in the bonded state as a counter ion to the composite (H1) or may be present in a solution containing the composite (H1). The method of removing the non-target substance (G2) is described later.

(2) Composite separation step

Next, the composite (H1) is separated from the sample (F1) by a magnetic force. The composite (H1) contains the core-shell particles (C), and the core-shell particles (C) contain the magnetic metal oxide particles (A). Thus, the composite (H1) can be collected by a magnetic force. Subsequently, the remaining sample (F1) is removed, whereby the composite (H1) can be separated from the sample (F1).

Such a separation method may be, for example, one that includes collecting the composite (H1) by a magnetic force of a magnet or the like from the outside of a reaction vessel, discharging the supernatant, and separating the composite (H1).

(3) Target substance dissociation step

Next, the target substance (G1) is obtained by dissociating the target substance (G1) from the composite (H1) .

The target substance (G1) may be dissociated from the composite (H1) by any method, such as one in which a substance that inhibits binding between the core-shell particles (C) and the target substance (G1) is added to dissociate the target substance (G1). The substance that inhibits binding between the core-shell particles (C) and the target substance (G1) varies depending on the types of the target substance (G1) and the substance (G). Examples thereof include substances that inhibit binding by the pH difference, salinity difference, or action of a surfactant. Examples of the substance capable of inhibiting binding (dissociation solution I) include water, ethanol, IPA, and an aqueous TE-HC1 buffer solution.

When the substance to be separated (G) is the non-target substance (G2), it is possible to use a separation and purification method including a composite formation step of forming a composite (H2) of the core-shell particles (C) and the non-target substance (G2) by contacting a sample (F2) containing the target substance (G1) and the non-target substance (G2) with the mixture (E); and a non-target substance removal step of removing the non-target substance (G2) from the sample (F2) by separating the composite (H2) from the sample (F2) by a magnetic force to obtain a sample (F3) containing the target substance (G1).

In this case, in the separation and purification method according to the second embodiment, the substance to be separated (G) is the non-target substance (G2), and it is a method of removing the non-target substance (G2) from the sample (F2) containing the non-target substance (G2).

The separation and purification method includes (1) a composite formation step and (2) a non-target substance removal step.

Each step is described below.

(1) Composite formation step

In this step, the sample (F) containing the target substance (G1) and the non-target substance (G2) is contacted with the mixture (E) of the core-shell particles (C) and the chaotropic salt (D) to form a composite (H1) of the core-shell particles (C) and the target substance (G1) and a composite (H2) of the core-shell particles (C) and the non-target substance (G2).

The composite (H2) may be formed by direct binding of the non-target substance (G2) to the core-shell particles (C). Alternatively, the core-shell particles (C) may contain the substance (G) that binds to the non-target substance (G2), and the composite (H2) may be formed by binding of the non-target substance (G2) to the core-shell particles (C) via the substance (G). The chaotropic salt (D) may be present in the bonded state as a counter ion to the composite (H1) and the composite (H2) or may be present in a solution containing the composite (H1) and the composite (H2).

(2) Non-target substance removal step

Next, the composite (H1) and the composite (H2) are separated from the sample (F2) by a magnetic force. The composite (H1) and the composite (H2) contain the core-shell particles (C), and the core-shell particles (C) contain the magnetic metal oxide particles (A). Thus, the composite (H1) and the composite (H2) can be collected by a magnetic force. In the composite (H2), the non-target substance (G2) exhibits weaker adsorption than the target substance (G1) onto the core-shell particles (C), and the non-target substance (G2) is dissociated from the composite (H2) and moves into a sample solution during magnetic collection. The composite (H1) and the composite (H2) are collected by a magnetic force of a magnet or the like from the outside of a reaction vessel. Then, the sample solution is stirred and the supernatant is discharged. Thus, the composite (H1) is magnetically collected from the outside of the reaction vessel and remains as the residue in the reaction vessel, while the non-target substance (G2) forming the composite (H2) is contained in the supernatant. The non-target substance (G2) can be separated by this method.

EXAMPLES

Production Example 1: Production of core-shell particles

(C1-1)

<Production of magnetic metal oxide particles (A)>

A reaction vessel was charged with iron (III) chloride hexahydrate (186 parts), iron (II) chloride tetrahydrate (68 parts), and water (1288 parts). These components were dissolved and the solution was heated to 50°C. While the temperature was maintained at 50°C to 55°C under stirring, 25 wt% ammonia water (280 parts) was added dropwise to the solution over one hour. Thus, magnetite particles were obtained in the water. Oleic acid (64 parts) as a dispersant was added to the resulting magnetite particles, and stirring was continued for two hours. After cooling to room temperature, the mixture was subjected to solid-liquid separation by decantation to obtain magnetite particles with oleic acid adsorbed thereon, which were then washed with water (1000 parts) three times and then washed with acetone (1000 parts) twice, followed by drying at 40°C for two days. Thus, magnetic metal oxide particles (A-1) having a volume average particle size of 15 nm were obtained.

<Production of core particle (P)>

Tetraethoxysilane (240 parts) was added to disperse the magnetic metal oxide particles (A-1) (80 parts) obtained in Production Method 1 to prepare a dispersion (B1). Next, a reaction vessel was charged with water (5050 parts), a 25 wt% aqueous ammonia solution (3500 parts), and Emalmin 200 (Sanyo Chemical Industries, Ltd.) (400 parts), and these components were mixed using Clearmix (M Technique Co., Ltd.) to obtain a solution (B2). After heating to 50°C, the dispersion (B1) was added dropwise to the solution (B2) over one hour under stirring with Clearmix at a rotation speed of 6000 rpm, followed by reaction at 50°C for one hour. After the reaction, the supernatant containing fine particles was removed by centrifugation at 2000 rpm for 20 minutes. Thus, core particles (P-1) containing the magnetic metal oxide particles (A-1) in an amount of 83 wt% were obtained.

<Production of core-shell particles (PC)>

A reaction vessel was charged with the core particles (P-1) (80 parts), deionized water (2500 parts), a 25 wt% aqueous ammonia solution (260 parts), ethanol (2500 parts), and tetraethoxysilane (1200 parts), and these components were mixed using Clearmix (M Technique). The mixture was reacted for two hours under stirring with Clearmix at a rotation speed of 6000 rpm. After the reaction, the supernatant containing fine particles was removed by centrifugation at 2000 rpm for 20 minutes. Deionized water (4000 parts) was added to the precipitated particles after centrifugation to re-disperse the particles. The dispersed particles were magnetically collected by contact with a magnet from the outside of the vessel, and the supernatant was removed. This operation was repeated 10 times. Thus, core-shell particles (PC-1) were obtained.

<Classification step of core-shell particles (PC)>

Water (5000 parts) was added to a solid phase containing the resulting core-shell particles (PC-1) to disperse the particles. The dispersion was centrifuged at 2800 rpm for one minute, and then the supernatant containing fine particles was removed. This operation was repeated four times (centrifugation step 1).
Subsequently, water (5000 parts) was added to the resulting solid phase to disperse the particles, followed by centrifugation at 600 rpm for one minute to collect the supernatant, whereby large-sized precipitated particles were removed. This operation was performed once (centrifugation step 2).
Further, the particles were magnetically collected using a magnet, and the supernatant was removed. Subsequently, after water (5000 parts) was added to disperse the core-shell particles, the particles were magnetically collected using a magnet, and the supernatant was removed. This operation was repeated 10 times (washing step 1). Thus, coreshell particles (C-1) were obtained.

Production Examples 2 to 5: Production of core-shell particles (C-2) to (C-5)

Similar operations as in Production Example 1 were carried out, except that the production conditions for <Classification step of core-shell particles (PC)> of Production Example 1 were changed as described in Table 1, whereby core-shell particles (C-2) to (C-5) were obtained.

Production Example 6: Production of core-shell particles (C-6)

Similar operations as in Production Example 1 were performed, except that the amount of iron (III) chloride hexahydrate was changed from 186 parts to 50 parts and the amount of iron (II) chloride tetrahydrate was changed from 68 parts to 18 parts in <Production of magnetic metal oxide particles (A)> of Production Example 1, whereby magnetic metal oxide particles (A-2) having an volume average particle size of 2 nm were obtained.

Subsequently, similar operations as in Production Example 1 were carried out for <Production of core particle (P)> and <Production of core-shell particles (PC)>, except that the production conditions for <Classification step of core-shell particles (PC)> were changed as described in Table 1, whereby core-shell particles (C-6) were obtained.

Production Example 7: Production of core-shell particles (C-7)

Similar operations as in Production Example 1 were performed, except that the amount of iron (III) chloride hexahydrate was changed from 186 parts to 583 parts and the amount of iron (II) chloride tetrahydrate was changed from 68 parts to 213 parts in <Production of magnetic metal oxide particles (A)> of Production Example 1, whereby magnetic metal oxide particles (A-3) having an volume average particle size of 47 nm were obtained.

Subsequently, similar operations as in Production Example 1 were carried out for <Production of core particle (P)> and <Production of core-shell particles (PC)>, except that the production conditions for <Classification step of core-shell particles (PC)> were changed as described in Table 1, whereby core-shell particles (C-7) were obtained.

Production Example 8: Production of core-shell particles (C-8)

Similar operations as in Production Example 1 were carried out, except that the amount of tetraethoxysilane was changed from 1200 parts to 20 parts in <Production of core-shell particles (PC)> of Production Example 1 and that the production conditions for <Classification step of core-shell particles (PC)> were changed as described in Table 1, whereby core-shell particles (C-8) were obtained.

Production Example 9: Production of core-shell particles (C-9)

Similar operations as in Production Example 1 were carried out, except that the amount of tetraethoxysilane was changed from 1200 parts to 30000 parts in <Production of core-shell particles (PC)> of Production Example 1 and that the production conditions for <Classification step of core-shell particles (PC)> were changed as described in Table 1, whereby core-shell particles (C-9) were obtained.

Production Example 10: Production of magnetic particles (c1) consisting of core particles

<Production of magnetic particles (Pc)>

Tetraethoxysilane (240 parts) was added to disperse the magnetic metal oxide particles (A-1) (80 parts) obtained in Production Example 1 to prepare the dispersion (B1). Next, a reaction vessel was charged with water (5050 parts), a 25 wt% aqueous ammonia solution (3500 parts), and Emalmin 200 (Sanyo Chemical Industries, Ltd.) (400 parts), and these components were mixed using Clearmix (M Technique Co., Ltd.) to obtain the solution (B2). After heating to 50°C, the dispersion (B1) was added dropwise to the solution (B2) over one hour under stirring with Clearmix at a rotation speed of 6000 rpm, followed by reaction at 50°C for one hour. After the reaction, the supernatant containing fine particles was removed by centrifugation at 2000 rpm for 20 minutes. Thus, a solid phase containing magnetic particles (Pc1) containing the magnetic metal oxide particles (A-1) in an amount of 83 wt% was obtained.

<Classification step of magnetic particles (Pc)>

Water (5000 parts) was added to the resulting solid phase containing the magnetic particles (Pc1) to disperse the particles. The dispersion was centrifuged at 2800 rpm for one minute, and then the supernatant containing fine particles was removed. This operation was repeated four times (centrifugation step 1).

Subsequently, water (5000 parts) was added to the resulting solid phase to disperse the particles, followed by centrifugation at 600 rpm for one minute to collect the supernatant, whereby precipitated large-sized particles were removed. This operation was performed once (centrifugation step 2).

Further, the particles were magnetically collected with a magnet, and the supernatant was removed. Subsequently, after water (5000 parts) was added to disperse the core-shell particles, the particles were magnetically collected with a magnet, and the supernatant was removed. This operation was repeated 10 times (washing step 1). Thus, non-core-shell magnetic particles (c1) consisting of core particles were obtained.

Comparative Production Example 1: Production of comparative particles (C'-1)

Similar operations as in Production Example 1 were carried out, except that NSA-17 (Sanyo Chemical Industries, Ltd.) was used instead of Emalmin 200 in <Production of core particles (P)> of Production Example 1 and that the production conditions for <Classification step of core-shell particles (PC)> of Production Example 1 were changed as described in Table 1, whereby comparative particles (C'-1) were obtained.

Comparative Production Example 2: Production of comparative particles (C'-2)

Similar operations as in Production Example 10 were carried out, except that the conditions for <Classification step of core-shell particles (Pc)> of Production Example 10 were changed as described in Table 1, whereby comparative particles (C'-2) were obtained.

The core-shell particles (C-1) to (C-9) and the non-core-shell magnetic particles (c1) respectively obtained in Production Examples 1 to 10 and the comparative particles (C'-1)and (C'-2) respectively obtained in Comparative Production Examples 1 and 2 were evaluated as follows.

<Method of measuring volume average particle size of magnetic metal oxide particles (A)>

Any 200 magnetic metal oxide particles (A) were observed using a scanning electron microscope (model number: JSM-7000F, manufacturer name: JEOL Ltd.) to measure the particle size, and the volume average particle size was determined. Table 1 shows the results.

<Method of measuring weight percentage of magnetic metal oxide particles (A) in core particles (P)>

For Production Examples 1 to 9 and Comparative Production Example 1 in which the core-shell particles were produced, any 20 core particles (P) obtained in <Production of core particles (P)> were each observed using a scanning electron microscope (model number: JSM-7000F, manufacturer name: JEOL Ltd.), and the amount of the magnetic metal oxide particle (A) in each of the core particles (P) was measured using an energy dispersive X-ray spectrometer (model number: INCA Wave/Energy; manufacturer name: Oxford Instruments). The average thereof was regarded as the amount S. The amount of silica was also measured in a similar manner, and the average thereof was regarded as the amount T. The weight percentage of the magnetic metal oxide particles (A) was determined by the following calculation formula. Table 1 shows the results.

```
Weight percentage (wt%) of magnetic metal oxide particles
(A) = [(S)/(S + T)] × 100
```

For Production Example 10 and Comparative Production Example 2 in which the non-core-shell magnetic particles consisting of the core particles were produced, any 20 magnetic particles (Pc1) obtained by the operation in <Production of magnetic particles (Pc)> were selected to measure the amount of the magnetic metal oxide particles (A) in the magnetic particles (Pc) in a similar manner. Table 1 shows the resulting values in the column titled "Weight percentage of magnetic metal oxide particles (A) in core particles (P)". In Production Example 10 and Comparative Production Example 2, each magnetic particle (Pc) is the core particle (P).

<Methods of measuring volume average particle size and coefficient of variation of core-shell particles (C-1) to (C-9), non-core-shell magnetic particles (c1), and comparative particles (C'-1)and (C'-2)>

The core-shell particles (C-1) to (C-9), the non-core-shell magnetic particles (c1), and the comparative particles (C'-1)and (C'-2) were separately dispersed in phosphate buffer solutions. Using he resulting dispersions of the magnetic particles as samples, the particle size distribution of each sample was measured by a laser diffraction/scattering particle size distribution measuring device ("Microtrac MT3300" from MicrotracBEL Corp.), and the volume average particle size and the coefficient of variation were calculated. Table 1 shows the results.

<Method of measuring average thickness of shell layers (Q) of core-shell particles (C-1) to (C-9) and comparative particles (C'-1)>

The core-shell particles (C-1) to (C-9) and the comparative particles (C'-1)obtained by <Classification step of core-shell particles (PC)> in respective Production Examples 1 to 9 and Comparative Production Example 1 were embedded in an epoxy resin. A microtome cross section of each particle was observed by a transmission electron microscope (model number "H-7100", Hitachi, Ltd.) to determine the thickness of the shell layer (Q) of the single core-shell particle (C) (or the comparative particle (C')) from the average of the thinnest and thickest portions thereof.

Further, the thickness of each of any 99 core-shell particles (C) (or comparative particles (C')) was determined in a similar manner as described above, and the average of a total of 100 particles was regarded as the average thickness of the shell layers (Q). Table 1 shows the results.

The resulting average thickness of the shell layers (Q) and the volume average particle size of the core-shell particles (C) (or the comparative particles (C')) were substituted into the following formula to calculate the average particle size of the core particles (P). Table 1 shows the ratio of the average thickness of the shell layers (Q) to the particle size of the core particle (P). Particle size of core particle (P) = (Volume average particle size of core-shell particles (C)) - 2 × (Average thickness of shell layers (Q))

[Table 1]

| | | | Production Example | | | | | | | | | | Comparative Production Example | |
|---|---|---|---|---|---|---|---|---|---|---|---|---|---|---|
| | | | 1 | 2 | 3 | 4 | 5 | 6 | 7 | 8 | 9 | 10 | 1 | 2 |
| | Magnetic particles | | Core-shell particles (C-1) | Core-shell particles (C-2) | Core-shell particles (C-3) | Core-shell particles (C-4) | Core-shell particles (C-5) | Core-shell particles (C-6) | Core-shell particles (C-7) | Core-shell particles (C-8) | Core-shell particles (C-9) | Non-core-shell magnetic particles (c1) | Core-shell particles (C'-1) | Non-core-shell magnetic particles (C'-2) |
| Production conditions | Surfactant used in production of core particle (P) | | Emalmin 200 | Emalmin 200 | Emalmin 200 | Emalmin 200 | Emalmin 200 | Emalmin 200 | Emalmin 200 | Emalmin 200 | Emalmin 200 | Emalmin 200 | NSA-17 | Emalmin 200 |
| | Classification step — Centrifugation step 1 | Rotation speed (rpm) | 2800 | 2800 | 1600 | 1400 | 1400 | 1600 | 1600 | 1600 | 1600 | 1600 | 600 | 2800 |
| | | Centrifugation time (min) | 1 | 1 | 1 | 1 | 1 | 1 | 1 | 1 | 1 | 1 | 10 | 1 |
| | | Number of repetitions | 4 | 20 | 20 | 20 | 40 | 20 | 20 | 20 | 20 | 20 | 20 | 4 |
| | Centrifugation step 2 | Rotation speed (rpm) | 600 | 600 | 800 | 1000 | 1300 | 800 | 800 | 800 | 800 | 800 | 300 | 600 |
| | | Centrifugation time (min) | 1 | 1 | 1 | 1 | 1 | 1 | 1 | 1 | 1 | 1 | 10 | 1 |
| | | Number of repetitions | 1 | 1 | 1 | 1 | 1 | 1 | 1 | 1 | 1 | 1 | 1 | 1 |
| | Washing step 1 | Number of repetitions | 10 | 10 | 10 | 10 | 10 | 10 | 10 | 10 | 10 | 10 | 10 | 10 |
| Magnetic metal oxide particles (A) | Volume average particle size (nm) of magnetic metal oxide particles (A) | | 15 | 15 | 15 | 15 | 15 | 2 | 47 | 15 | 15 | 15 | 15 | 15 |
| Properties of magnetic particles (core-shell particles (C) or non-core-shell magnetic particles (c)) | Weight percentage of magnetic metal oxide particles (A) in core particles (P) (wt%) | | 83 | 83 | 83 | 83 | 83 | 83 | 83 | 83 | 83 | 83 | 83 | 83 |
| | Volume average particle size (μm) of magnetic particles | | 2.1 | 2.2 | 2.2 | 2.0 | 1.9 | 2.2 | 2.2 | 2.1 | 2.2 | 1.9 | 2 | 1.9 |
| | Coefficient of variation (C.V) of magnetic particles | | 47% | 32% | 21% | 11% | 6% | 21% | 21% | 21% | 21% | 20% | 65% | 61% |
| | Average thickness (nm) of shell layers (Q) | | 100 | 100 | 100 | 100 | 100 | 100 | 100 | 5 | 2900 | No shell layer | 100 | No shell layer |
| | Ratio of average thickness of shell layers (Q) to particle size of core particle (P) | | 0.053 | 0.050 | 0.050 | 0.056 | 0.059 | 0.049 | 0.048 | 0.003 | 1.520 | No shell layer | 0.056 | No shell layer |

<Examples 1 to 13 and Comparative Examples 1 to 3: Production of magnetic particle composition (e)>

(1) For each type of the core-shell particles (C-1) to (C-9) and the non-core-shell magnetic particles (c1) respectively obtained in Production Examples 1 to 10 and the comparative particles (C'-1)and (C'-2) respectively obtained in Comparative Production Examples 1 and 2, the particles (296 mg) were added to a glass container, and purified water (10 mL) was added to the glass container, followed by stirring in a vortex mixer, whereby a water dispersion containing magnetic particles was obtained.

(2) The water dispersion (600 $\mu$L) containing the magnetic particles (weight of the magnetic particles in the dispersion: 177.6 mg) was placed in a 15-mL microtube. The magnetic particles were immobilized with a magnet to a wall surface of the microtube by placing the magnet on the microtube from the outside, and only the dispersion medium (water) of the water dispersion was discharged. An aqueous chaotropic salt solution (900 $\mu$L) was added to the microtube containing only the magnetic particles. Thus, magnetic particle compositions (e1) to (e13) respectively according to Examples 1 to 13 and comparative magnetic particle compositions (e'1) and (e'2) respectively according to Comparative Examples 1 and 2 were produced.

The water dispersion (600 $\mu$L) of the core-shell particles (C-3) produced in (1) was placed in a 15-mL microtube. The magnetic particles were immobilized with a magnet to a wall surface of the microtube by placing the magnet on the microtube from the outside, and only the dispersion medium (water) of the water dispersion was discharged. Purified water (900 $\mu$L) was added to the microtube containing only the magnetic particles. Thus, a comparative magnetic particle composition (e'3) according to Comparative Example 3 was produced.

The aqueous chaotropic salt solution was an aqueous solution obtained by dissolving the chaotropic salt (D) described in Table 2 to give a concentration of 6 M in Tris-EDTA buffer (tris(hydroxymethyl)aminomethane: 10 mM; tetrasodium ethylenediaminetetraacetate: 2 mM; pH 7.86).

[Table 2]

| | Example | | | | | | | | | | | | | Comparative Example | | |
|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 1 | 2 | 3 |
| Magnetic particle composition | e1 | e2 | e3 | e4 | e5 | e6 | e7 | e8 | e9 | e10 | e11 | e12 | e13 | e'1 | e'2 | e'3 |
| Magnetic particles | Core-shell particles (C-1) | Core-shell particles (C-2) | Core-shell particles (C-3) | Core-shell particles (C-4) | Core-shell particles (C-5) | Core-shell particles (C-6) | Core-shell particles (C-7) | Core-shell particles (C-8) | Core-shell particles (C-9) | Core-shell particles (C-3) | Non-core-shell magnetic particles (c1) | Core-shell particles (C-3) | Core-shell particles (C-3) | Core-shell particles (C'-1) | Non-core-shell magnetic particles (C'-2) | Core-shell particles (C-3) |
| Chaotropic salt | Guanidinium thiocyanate | Guanidinium thiocyanate | Guanidinium thiocyanate | Guanidinium thiocyanate | Guanidinium thiocyanate | Guanidinium thiocyanate | Guanidinium thiocyanate | Guanidinium thiocyanate | Guanidinium thiocyanate | Guanidinium thiocyanate | Guanidinium thiocyanate | Guanidine hydrochloride | Sodium perchlorate | Guanidinium thiocyanate | Guanidinium thiocyanate | Absent |
| Weight ratio of magnetic particles to chaotropic salt | 2/98 | 2/98 | 2/98 | 2/98 | 2/98 | 2/98 | 2/98 | 2/98 | 2/98 | 16/84 | 2/98 | 4/96 | 3/97 | 2/98 | 2/98 | - |

<Examples 14 to 30 and Comparative Examples 4 to 6: Separation of DNA>

<Composite formation step>

(1) To provide a sample (F1) containing DNA (a target substance (D1)) and BSA (impurities), a mixture of an aqueous DNA solution (150 μL) and an aqueous BSA solution (150 μL) was prepared.

The aqueous DNA solution was an aqueous solution obtained by dissolving DNA (deoxyribonucleic acid derived from salmon semen available from FUJIFILM Wako Pure Chemical Corporation) at a concentration of 2.40 mg/ml in the Tris-EDTA buffer. The aqueous BSA solution was an aqueous solution obtained by dissolving BSA at a concentration of 2.40 mg/ml in the Tris-EDTA buffer.

(2-1) The sample (F1) (300 μL) was added to each of the microtubes separately containing the magnetic particle compositions (e1) to (e13) respectively obtained in Examples 1 to 13 and the comparative magnetic particle compositions (e'1) to (e'3) respectively obtained in Comparative Examples 1 to 3. Subsequently, each microtube was shaken in a shaker incubator (37°C, 350 rpm, 2.0 hr) to promote contact of the magnetic particles with DNA and BSA so as to a form a composite of the magnetic particles, DNA, and BSA. Thus, dispersions separately containing composites for DNA separation (1-1) to (1-13) respectively according to Examples 14 to 26 and composites for DNA separation (H1-1) to (H1-3) respectively according to Comparative Examples 4 to 6 described in Table 3 were obtained.

(2-2) The sample (F1) (300 μL) was added to a microtube containing the magnetic particle composition (e11) obtained in Example 11, and further, 99.5% ethanol (available from FUJIFILM Wako Pure Chemical Corporation) (900 μL) was added thereto. Subsequently, the microtube was shaken in a shaker incubator (37°C, 350 rpm, 2.0 hr) to promote contact of the magnetic particles with DNA and BSA so as to form a composite of the magnetic particles, DNA, and BSA. Thus, a dispersion containing a composite for DNA separation (1-14) according to Example 27 was obtained. In Example 27, the ethanol concentration (volume fraction) in the contents of the microtube was 43 vol% based on the volume of liquid components among the components constituting the contents of the microtube.

(2-3) The sample (F1) (300 μL) was added to a microtube containing the magnetic particle composition (e3) obtained in Example 3, and further, 99.5% ethanol (900 μL, 300 μL, or 1800 μL) was added thereto. Subsequently, the microtube was shaken in a shaker incubator (37°C, 350 rpm, 2.0 hr) to promote contact of the magnetic particles with DNA and BSA so as to form a composite of the magnetic particles, DNA, and BSA. Thus, dispersions separately containing composites for DNA separation (1-15) to (1-17) respectively according to Examples 28 to 30 were obtained. In Examples 28, 29, and 30, the ethanol concentrations (volume fraction) in the contents of the microtubes were respectively 43, 20, and 60 vol%, based on the volumes of liquid components among the components constituting the contents of the respective microtubes.

(3) After the formation of the composites for DNA separation (1-1) to (1-17) and (H1-1) to (H1-3), each microtube was allowed to stand to separate a precipitate from the supernatant, and the supernatant was removed to collect the precipitate containing the composite for DNA separation.

<Composite separation step>

(4) A 70 vol% aqueous ethanol solution (900 μL) was added to each collected precipitate to prepare a dispersion, and a magnet was placed on a container containing the dispersion from the outside to collect a composite for DNA separation on the magnet side. While the composite for DNA separation was collected on the magnet side, the supernatant was removed, and a 70 vol% aqueous ethanol solution (900 μL) was added again to prepare a dispersion. Dispersion in the 70 vol% aqueous ethanol solution and removal of the supernatant were repeated 10 times in total, whereby the composite for DNA separation was collected in the microtube.

<Target substance dissociation step>

(5) The Tris-EDTA Buffer (400 μL) was added to the microtube containing the collected composite for DNA separation, which was obtained in (4) above. The mixture was stirred for 15 seconds every five minutes in a vortex mixer. This operation was repeated three times. Thus, DNA and BSA were dissociated from the composite for DNA separation.

Subsequently, a magnet was placed on the microtube from the outside to collect the magnetic particles on the magnet side, and the whole supernatant (the entire 400 μL) containing the separated DNA and BSA was collected with a pipette.

<Measurement of collected DNA and BSA>

<Pre-measurement treatment>

An amount of 100 μL was sampled from the supernatant (400 μL) collected in <Target substance dissociation step> in (5) above. The sample was added dropwise to a desalting and buffer exchange gravity column (PD-10 available from GE Healthcare), and DNA and BSA in the supernatant were adsorbed onto the adsorbent.

Subsequently, purified water (1,100 μL) as an eluent of the adsorbed DNA and BSA was added to the column, and an eluate discharged by gravity flow was collected in a microtube. The microtube was replaced upon collection of 1,100 μL of eluate. Again, purified water (1,100 μL) was added to the column, and 1,100 μL of eluate was collected in the microtube. The operation of replacing the microtube and adding purified water (1,100 μL) to the column to collect 1,100 μL of eluate in the microtube was repeated additional four times. The operation of collecting 1,100 μL of eluate in the microtube was repeated a total of six times.

A portion (200 μL) was extracted from each of the eluates collected in the third to sixth operation out of the total six operations of collecting 1,100 μL of eluate in the microtube, and these eluates were mixed at equal volume to obtain a solution (a total of 800 μL) as a "DNA and BSA measurement sample".

<Measurement of amount of collected DNA>

DNA solutions of known concentrations were prepared as standard solutions, and the absorbance at 260 nm was measured using a spectrophotometer to produce a calibration curve showing the relationship between the absorbance and the DNA concentration.

The absorbance of the "DNA and BSA measurement sample" at 260 nm was also measured, and the DNA concentration in the eluate obtained by the pre-treatment was determined using the calibration curve.

The weight of DNA in the supernatant (100 μL) added to the column and collected in <Target substance dissociation step> was calculated from the DNA concentration in the eluate obtained by the pre-treatment, and further, the weight of DNA in the whole supernatant collected in <Target substance dissociation step> was calculated.

The amount of the collected DNA is affected by the surface area of the magnetic particles. Thus, for each of the examples and the comparative examples, a value (the weight of the collected DNA per unit surface area of the magnetic particles) was determined by dividing the weight of DNA in the whole supernatant by the total surface area of the magnetic particles used in the corresponding example or comparative example. Table 3 shows the resulting values.

<Measurement of amount of collected BSA>

Using BSA (bovine serum-derived albumin at low salt concentration available from FUJIFILM Wako Pure Chemical Corporation) standard solutions of known concentrations, the absorbance at 280 nm was measured by a spectrophotometer to produce a calibration curve showing the relationship between the absorbance and the BSA concentration.

The absorbance of the "DNA and BSA measurement sample" at 280 nm was also measured, and the BSA concentration in the eluate obtained by the pre-treatment of the column was determined using the calibration curve.

This value was used as in <Measurement of DNA concentration> to determine a value (the weight of collected BSA per unit surface area of magnetic particles) by dividing the weight of BSA in the whole supernatant by the total surface area of the magnetic particles used in the corresponding example. Table 3 shows the resulting values.

<Measurement of purity of DNA>

Using ultraviolet-visible spectrophotometer "UV-1800" (Shimadzu Corporation), the absorbance (optical path length: 10 mm) of the DNA and BSA measurement sample at 280 nm and the absorbance (optical path length: 10 mm) thereof at 260 nm were measured.

A value of the ratio of absorbance at 260 nm to absorbance at 280 nm (absorbance ratio = A260/A280) was calculated and used as an index of DNA purity for evaluation. Table 3 shows the results.

100% purity DNA has an absorbance ratio (A260/A280) of 1.8. Thus, the purity of the collected DNA is determined to be high when the absorbance ratio is in the range of 1.76 to 1.90.

[Table 3]

| | Example | | | | | | | | | | | | | | | | | Comparative Example | | |
|---|---|---|---|---|---|---|---|---|---|---|---|---|---|---|---|---|---|---|---|---|
| | 14 | 15 | 16 | 17 | 18 | 19 | 20 | 21 | 22 | 23 | 24 | 25 | 26 | 27 | 28 | 29 | 30 | 4 | 5 | 6 |
| Composite for DNA separation | 1-1 | 1-2 | 1-3 | 1-4 | 1-5 | 1-6 | 1-7 | 1-8 | 1-9 | 1-10 | 1-11 | 1-12 | 1-13 | 1-14 | 1-15 | 1-16 | 1-17 | H1-1 | H1-2 | H1-3 |
| Magnetic particle composition | e1 | e2 | e3 | e4 | e5 | e6 | e7 | e8 | e9 | e10 | e11 | e12 | e13 | e11 | e3 | e3 | e3 | e'1 | e'2 | e'3 |
| Ethanol concentraion (vol%) | 0 | 0 | 0 | 0 | 0 | 0 | 0 | 0 | 0 | 0 | 0 | 0 | 0 | 43 | 43 | 20 | 60 | 0 | 0 | 0 |
| Total surface area of magnetic particles ($m^2$) | 0.018 | 0.016 | 0.016 | 0.020 | 0.022 | 0.018 | 0.018 | 0.020 | 0.003 | 0.016 | 0.024 | 0.016 | 0.016 | 0.024 | 0.016 | 0.016 | 0.016 | 0.072 | 0.024 | 0.016 |
| Amount of recovered DNA ($\mu g/m^2$) | 372 | 440 | 464 | 363 | 335 | 330 | 298 | 309 | 270 | 248 | 230 | 261 | 208 | 1038 | 3195 | 472 | 919 | 204 | 174 | 186 |
| Amount of recovered BSA ($\mu g/m^2$) | 68.0 | 18.0 | 7.0 | 55.0 | 53.0 | 29.7 | 50.5 | 16.0 | 30.0 | 24.2 | 10.0 | 8.0 | 32.0 | 13.0 | 11.0 | 12 | 24 | 48.2 | 39 | 15 |
| Purity of DNA (A260/A280) | 1.79 | 1.79 | 1.80 | 1.79 | 1.79 | 1.81 | 1.79 | 1.81 | 1.83 | 1.78 | 1.81 | 1.75 | 1.78 | 1.79 | 1.84 | 1.81 | 1.78 | 1.74 | 1.92 | 1.68 |

<Examples 31 to 41 and Comparative Examples 7 to 9: Separation of DNA from environmental sample>

<Composite formation step>

(1) Solutions of collected DNA, which were separately extracted from potting mix, collected soil, river water, and animal excrement, were each prepared as a sample (F2).
The potting mix and collected soil are examples of soil.
Then, according to the combinations shown in Table 4, the sample (F2) (300 μL) was added to each of the microtubes containing the respective magnetic particle compositions (e1) to (e13) respectively obtained in Examples 1 to 13 and the comparative compositions (e'1) to (e'3) respectively obtained in Comparative Example 1 to 3. For Examples 40 and 41, 99.5% ethanol (900 μL) was also added to each microtube.
Each microtube containing the contents was shaken in a shaker incubator (37°C, 350 rpm, 2.0 hr) to form a composite of the magnetic particles and DNA (a composite for DNA separation 2). Thus, composites for DNA separation 2 (2-1) to (2-11) respectively according to Examples 31 to 41 and composites for DNA separation 2 (H2-1) to (H2-3) respectively according to Comparative Examples 7 to 9 described in Table 4 were formed.
(2) After the formation of the composites for DNA separation 2, each microtube was allowed to stand to separate a precipitate from the supernatant, and the supernatant was removed to collect the precipitate containing the corresponding composite for DNA separation 2.

<Composite separation step>

(3) A 70 vol% aqueous ethanol solution (900 μL) was added to each collected precipitate to prepare a dispersion, and a magnet was placed on a container containing the dispersion from the outside to collect the composite for DNA separation 2 on the magnet side. While the composite for DNA separation 2 was collected on the magnet side, the supernatant was removed, and a 70 vol% aqueous ethanol solution (900 μL) was added again to prepare a dispersion. Dispersion in the 70 vol% aqueous ethanol solution and removal of the supernatant were repeated 10 times in total, whereby each composite for DNA separation 2 was collected in the microtube.

<Target substance dissociation step>

(4) The Tris-EDTA Buffer (400 μL) was added to each microtube containing the collected composite for DNA separation 2, which was obtained in (3) above. The mixture was stirred for 15 seconds every five minutes in a vortex mixer. This operation was repeated three times. Thus, DNA was dissociated from the composite for DNA separation 2.
Subsequently, a magnet was placed on the microtube from the outside to collect the magnetic particles on the magnet side, and the whole supernatant (the entire 400 μL) containing the separated DNA was collected with a pipette.

<Analysis of collected DNA>

Each supernatant collected in <Target substance dissociation step> for the composite for DNA separation 2 was subjected to <Pre-measurement treatment> as in the supernatant collected in <Target substance dissociation step> for the composite for DNA separation 1, whereby a DNA measurement sample was produced. Then, analysis was performed as in <Measurement of DNA concentration> and <Measurement of purity of DNA> for the composite for DNA separation 1. Table 4 shows the results.

<Production of collected DNA solution from potting mix>

To a Kenis plant box (available from AGC Techno Glass Co., Ltd.) with a 4 mm diameter hole on the bottom was added gardening potting mix sterilized by heat at 130°C for 30 minutes (product name: "Hana to yasai no tsuchi" available from Taiyo Shokusan, K.K.) (100 g). Then, two Komatsuna seeds (available from Takii & Co., Ltd.) were seeded in each of three places, and covered with soil (1 cm). The seeds were then allowed to stand in an artificial meteorological device (LPH-241/411SP available from Nippon Medical & Chemical Instruments Co., Ltd) set at a temperature of 20°C, a humidity of 55%, a light intensity of 60%, and a 12-hour light and 12-hour dark cycle. The seeds were nurtured by watering with tap water (50 ml) once every two days, and the potting mix was collected from the plant box after two weeks of cultivation from germination.
A portion (2 g in wet weight) was sampled from the collected potting mix, and suspended in 10 mM Tris-EDTA-HCl buffer ((tris(hydroxymethyl)aminomethane: 10 mM, tetrasodium ethylenediaminetetraacetate: 1 mM, pH 7.4) (2 ml), followed by shaking at 5°C for one hour.
Subsequently, sodium dodecyl sulfate (available from FUJIFILM Wako Pure Chemical Corporation) (20 μL)

was added to the suspension, followed by incubation at 65°C to 70°C for 30 minutes. Subsequently, centrifugation was performed at 3600 G at room temperature for 10 minutes. The supernatant was obtained as a collected DNA solution.

<Production of collected DNA solution from collected soil>

Soil (wet weight: 2 g) collected from outdoor environment (near Sanyo Chemical Industries, Ltd., Katsura Research Laboratory in Nishikyo-ku, Kyoto City, Kyoto, Japan) was suspended in 10 mM Tris-EDTA-HCl buffer (pH 7.4) (2 ml), followed by shaking at 5°C for one hour.

Subsequently, sodium dodecyl sulfate (20 μL) was added to the suspension, followed by incubation at 65°C to 70°C for 30 minutes. Subsequently, centrifugation was performed at 3600 G at room temperature for 10 minutes. The supernatant was obtained as a collected DNA solution.

<Production of collected DNA solution from river water>

River water (40 mL) collected from Katsura River (near Katsura Imperial Villa, Nishikyo-ku, Kyoto City, Kyoto, Japan) was filtered through a glass fiber filter having a pore diameter of 0.7 μm) (GF/F, Whatman). After the filtration, the filter was transferred to a microtube, and 10 mM Tris-EDTA-HCl buffer (pH 7.4) (3.5 ml) was added to the microtube, followed by stirring in a vortex mixer for five minutes.

Subsequently, protease K (available from Sigma Aldrich) (50 μg) and sodium dodecyl sulfate (17.5 μL) were added, followed by incubation at 56°C for 15 minutes. Subsequently, centrifugation was performed at 3600 G at room temperature for 10 minutes. The supernatant was obtained as a collected DNA solution.

<Production of collected DNA solution from animal excrement>

Feces (1 g) of raised C57BL/6 mice (available from Shimizu Laboratory Supplies Co., Ltd.) were suspended in a phosphate buffer (9 ml) and the suspension was allowed to stand. Then, the supernatant (2 ml) was collected, followed by centrifugation at 15000 rpm for three minutes. After the centrifugation, the supernatant was discarded. To the remaining precipitate was added a SDS solution obtained by dissolving sodium dodecyl sulfate at a concentration of 1% in 10 mM Tris-EDTA-HCl buffer (pH 7.4), followed by incubation at 65°C to 70°C for 30 minutes. Subsequently, centrifugation was performed at 3600 G at room temperature for 10 minutes. The supernatant was obtained as a collected DNA solution.

[Table 4]

| | Example | | | | | | | | | | | Comparative Example | | |
|---|---|---|---|---|---|---|---|---|---|---|---|---|---|---|
| | 31 | 32 | 33 | 34 | 35 | 36 | 37 | 38 | 39 | 40 | 41 | 7 | 8 | 9 |
| Magnetic particle composition | e1 | e3 | e5 | e3 | e3 | e3 | e12 | e13 | e11 | e11 | e3 | e'1 | e'2 | e'3 |
| Magnetic particles | Core-shell particles (C-1) | Core-shell partic les (C-3) | Core-shell partic les (C-5) | Core-shell partic les (C-3) | Core-shell partic les (C-3) | Core-shell partic les (C-3) | Core-shell partic les (C-3) | Core-shell partic les (C-3) | Non-core-shell magnetic partic les (c1) | Non-core-shell magnetic partic les (c1) | Core-shell particles (C-3) | Core-shell partic les (C'-1) | Non-core-shell magnetic partic les (C'-2) | Core-shell partic les (C-3) |
| Chaotropic salt | Guanidin-ium thiocy-anate | Guanidin-ium thiocy-anate | Guanidin-ium thiocy-anate | Guanidin-ium thiocy-anate | Guanidin-ium thiocy-anate | Guanidin-ium thiocy-anate | Guanidine hydrochlo-ride | Sodium perchlo-rate | Guanidin-ium thiocy-anate | Guanidin-ium thiocy-anate | Guanidin-ium thiocy-anate | Guanidinium thi-ocyanate | | Absent |
| Ethanol | Absent | | | | | | | | | Present | | Absent | | |
| Ethanol concentration (vol%) | 0 | 0 | 0 | 0 | 0 | 0 | 0 | 0 | 0 | 43 | 43 | 0 | 0 | 0 |
| Origin of re-covered DNA in sam-ple (F2) | Potting mix | | | Collected soil | River water | Animal excrement | Potting mix | | | | | Potting mix | | |
| Composite (C-DNA) | 2-1 | 2-2 | 2-3 | 2-4 | 2-5 | 2-6 | 2-7 | 2-8 | 2-9 | 2-10 | 2-11 | H2-1 | H2-2 | H2-3 |
| Amount of recovered DNA ($\mu$g/m$^2$) | 70 | 88 | 80 | 88 | 78 | 238 | 66 | 74 | 43 | 324 | 492 | 20 | 42 | 44 |
| Purity of DNA (A260/A280) | 1.76 | 1.81 | 1.85 | 1.81 | 1.81 | 1.81 | 1.81 | 1.77 | 1.78 | 1.76 | 1.80 | 2.25 | 1.64 | 1.70 |

EP 4 032 854 A1

<Examples 42 to 58 and Comparative Examples 10 to 12: Separation of RNA>

<Composite formation>

(1) To provide a sample (F3) containing RNA (a target substance (D2)) and BSA (impurities), a mixture of an aqueous BSA solution 150 µL and an aqueous RNA solution 150 µL in which citric acid (available from FUJIFILM Wako Pure Chemical Corporation) was dissolved at a concentration of 25 mM was prepared.

The aqueous RNA solution is an aqueous solution obtained by dissolving RNA (ribonucleic acid derived from yeast available from FUJIFILM Wako Pure Chemical Corporation) at a concentration of 2.40 mg/ml in the same Tris-EDTA Buffer used in Examples 14 to 26. The aqueous BSA solution is the same aqueous solution used in Examples 14 to 26.

(2-1) The sample (F3) (300 µL) was added to each of the microtubes separately containing the magnetic particle composition (e1) to (e13) respectively obtained in Examples 1 to 13 and the comparative magnetic particle composition (e'1) to (e'3) respectively obtained in Comparative Example 1 to 3. Subsequently, each microtube was shaken in a shaker incubator (37°C, 350 rpm, 2.0 hr) to promote contact of the magnetic particles with RNA and BSA so as to a form a composite of the magnetic particles, RNA, and BSA. Thus, dispersions separately containing composites for RNA separation (3-1) to (3-13) respectively according to Examples 42 to 54 and composites for RNA separation (H3-1) to (H3-3) respectively according to Comparative Examples 10 to 12 described in Table 5 were obtained.

(2-2) The sample (F3) (300 µL) was added to a microtube containing the magnetic particle composition (e11) obtained in Example 11, and further, 99.5% ethanol (available from FUJIFILM Wako Pure Chemical Corporation) (900 µL) was added thereto. Subsequently, the microtube was shaken in a shaker incubator (37°C, 350 rpm, 2.0 hr) to promote contact of the magnetic particles with RNA and BSA so as to form a composite of the magnetic particles, RNA, and BSA. Thus, a dispersion containing a composite for RNA separation (3-14) according to Example 55 was obtained. In Example 55, the ethanol concentration (volume fraction) in the contents of the microtube was 43 vol% based on the volume of liquid components among the components constituting the contents of the microtube.

(2-3) The sample (F3) (300 µL) was added to a microtube containing the magnetic particle composition (e3) obtained in Example 3, and further, 99.5% ethanol (900 µL, 300 µL, or 1800 µL) was added thereto. Subsequently, the microtube was shaken in a shaker incubator (37°C, 350 rpm, 2.0 hr) to promote contact of the magnetic particles with RNA and BSA so as to form a composite of the magnetic particles, RNA, and BSA. Thus, dispersions separately containing composites for RNA separation (3-15) to (3-17) respectively according to Examples 56 to 58 were obtained. In Examples 56, 57, and 58, the ethanol concentrations (volume fraction) in the contents of the microtubes were respectively 43, 20, and 60 vol%, based on the volumes of liquid components among the components constituting the contents of the respective microtubes.

(3) After the formation of the composites for RNA separation (3-1) to (3-17) and (H3-1) to (H3-3), each microtube was allowed to stand to separate a precipitate from the supernatant, and the supernatant was removed to collect the precipitate containing the composite for RNA separation.

<Composite separation step>

(4) A 70 vol% aqueous ethanol solution (900 µL) was added to each collected precipitate to prepare a dispersion, and a magnet was placed on a container containing the dispersion from the outside to collect a composite for RNA separation on the magnet side. While the composite for RNA separation was collected on the magnet side, the supernatant was removed, and a 70 vol% aqueous ethanol solution (900 µL) was added again to prepare a dispersion. Dispersion in the 70 vol% aqueous ethanol solution and removal of the supernatant were repeated 10 times in total, whereby the composite for RNA separation was collected in the microtube.

<Target substance dissociation step>

(5) The Tris-EDTA Buffer (400 µL) was added to the microtube containing the collected composite for RNA separation, which was obtained in (4) above. The mixture was stirred for 15 seconds every five minutes in a vortex mixer. This operation was repeated three times. Thus, RNA and BSA were dissociated from the composite for RNA separation.

Subsequently, a magnet was placed on the microtube from the outside to collect the magnetic particles on the magnet side, and the whole supernatant (the entire 400 µL) containing the separated RNA and BSA was collected with a pipette.

<Analysis of collected RNA and BSA>

The supernatant collected in <Target substance dissociation step> for the composite for RNA separation was subjected to <Pre-measurement treatment> as in the supernatant collected in <Target substance dissociation step> for the composite for DNA separation 1, whereby an RNA measurement sample was produced.

<Measurement of amount of collected RNA>

RNA solutions of known concentrations were prepared as standard solutions, and the absorbance at 260 nm was measured using a spectrophotometer to produce a calibration curve showing the relationship between the absorbance and the RNA concentration.

The absorbance of the "RNA measurement sample" at 260 nm was also measured, and the RNA concentration in the eluate obtained by the pre-treatment was determined using the calibration curve.

The amount of the collected RNA was determined by calculation in a similar manner as in the composite for DNA separation 1 from the RNA concentration in the eluate obtained by the pre-treatment. Table 5 shows the results.

<Measurement of amount of collected BSA>

Measurement and calculation were performed for the "RNA measurement sample" as in <Measurement of amount of collected BSA> for the composite for DNA separation 1 to determine the amount of collected BSA. Table 5 shows the results.

<Measurement of purity of RNA>

Measurement and calculation were performed for the "RNA measurement sample" as in <Measurement of purity of DNA> for the composite for DNA separation 1. Table 5 shows the results.

100% purity RNA has an absorbance ratio (A260/A280) of 2.0. Thus, the purity of RNA is determined to be high when the absorbance ratio is in the range of 1.95 to 2.05.

[Table 5]

| | Example | | | | | | | | | | | | | | | | | Comparative Example | | |
|---|---|---|---|---|---|---|---|---|---|---|---|---|---|---|---|---|---|---|---|---|
| | 42 | 43 | 44 | 45 | 46 | 47 | 48 | 49 | 50 | 51 | 52 | 53 | 54 | 55 | 56 | 57 | 58 | 10 | 11 | 12 |
| Composite (C-DNA) | 3-1 | 3-2 | 3-3 | 3-4 | 3-5 | 3-6 | 3-7 | 3-8 | 3-9 | 3-10 | 3-11 | 3-12 | 3-13 | 3-14 | 3-15 | 3-16 | 3-17 | H3-1 | H3-2 | H3-3 |
| Magnetic particle composition | e1 | e2 | e3 | e4 | e5 | e6 | e7 | e8 | e9 | e10 | e11 | e12 | e13 | e11 | e3 | e3 | e3 | e'1 | e'2 | e'3 |
| Magnetic particles | Core-shell particles (C-1) | Core-shell particles (C-2) | Core-shell particles (C-3) | Core-shell particles (C-4) | Core-shell particles (C-5) | Core-shell particles (C-6) | Core-shell particles (C-7) | Core-shell particles (C-8) | Core-shell particles (C-9) | Core-shell particles (C-3) | Non-core-shell magnetic particles (c1) | Core-shell particles (C-3) | Core-shell particles (C-3) | Non-core-shell magnetic particles (c1) | Core-shell particles (C-3) | Core-shell particles (C-3) | Core-shell particles (C1-3) | Core-shell particles (C'-1) | Non-core-shell magnetic particles (C'-2) | Core-shell particles (C-3) |
| Chaotropic salt | Guanidinium thiocyanate | Guanidinium thiocyanate | Guanidinium thiocyanate | Guanidinium thiocyanate | Guanidinium thiocyanate | Guanidinium thiocyanate | Guanidinium thiocyanate | Guanidinium thiocyanate | Guanidinium thiocyanate | Guanidinium thiocyanate | Guanidinium thiocyanate | Guanidine hydrochloride | Sodium perchlorate | Guanidinium thiocyanate | Guanidinium thiocyanate | Guanidinium thiocyanate | Guanidinium thiocyanate | Guanidinium thiocyanate | Guanidinium thiocyanate | Absent |
| Ethanol | Absent | | | | | | | | | | | | | Present | | | | Absent | | |
| Ethanol concentration (vol%) | 0 | 0 | 0 | 0 | 0 | 0 | 0 | 0 | 0 | 0 | 0 | 0 | 0 | 43 | 43 | 20 | 60 | 0 | 0 | 0 |
| Amount of recovered RNA ($\mu g/m^2$) | 200 | 232 | 282 | 274 | 271 | 242 | 230 | 255 | 218 | 224 | 135 | 204 | 188 | 630 | 1678 | 342 | 408 | 142 | 98 | 143 |
| Amount of recovered BSA ($\mu g/m^2$) | 52.0 | 49.2 | 38.2 | 43.0 | 40.2 | 35.0 | 38.8 | 34.1 | 32.5 | 29.7 | 37.2 | 12.2 | 22.0 | 13.0 | 39.1 | 38.2 | 36.6 | 48.1 | 34.4 | 25.2 |
| Purity of RNA (A260/A280) | 1.98 | 1.97 | 2.01 | 1.98 | 2.00 | 1.96 | 2.02 | 2.01 | 2.01 | 1.98 | 1.96 | 1.95 | 2.04 | 1.96 | 2.01 | 1.99 | 1.97 | 1.94 | 1.94 | 2.08 |

**Claims**

1.  A magnetic particle composition (e), comprising:

    magnetic particles (c); and
    a chaotropic salt (D),
    the magnetic particles (c) each including a core particle (P) that is a magnetic silica particle containing a magnetic metal oxide particle (A),
    wherein the magnetic metal oxide particle (A) in the core particle (P) has a weight percentage of 60 wt% or more based on the weight of the core particle (P), and
    the magnetic particles (c) have a particle size distribution with a coefficient of variation of 5 to 50%.

2.  The magnetic particle composition (e) according to claim 1,
    wherein a weight ratio (c/D) of the magnetic particles (c) to the chaotropic salt (D) is 2/98 to 16/84.

3.  The magnetic particle composition (e) according to claim 1 or 2,
    wherein the magnetic metal oxide particle (A) has a volume average particle size of 1 to 50 nm.

4.  The magnetic particle composition (e) according to any one of claims 1 to 3,
    wherein the magnetic metal oxide particle (A) contains iron oxide.

5.  The magnetic particle composition (e) according to any one of claims 1 to 4,
    wherein the magnetic particles (c) each consist of the core particle (P) that is a magnetic silica particle containing the magnetic metal oxide particle (A).

6.  The magnetic particle composition (e) according to any one of claims 1 to 4,

    wherein the magnetic particles (c) are core-shell particles (C) each including the core particle (P) that is a magnetic silica particle containing the magnetic metal oxide particle (A) and a shell layer (Q) that is a silica layer having an average thickness of 3 to 3000 nm formed on a surface of the core particle (P), and
    the magnetic particle composition (e) is a mixture (E) of the core-shell particles (C) and the chaotropic salt (D) .

7.  The magnetic particle composition (e) according to claim 6,
    wherein the core-shell particles (C) have a volume average particle size of 0.5 to 20 $\mu$m.

8.  The magnetic particle composition (e) according to any one of claims 1 to 7,
    wherein the magnetic particle composition (e) is for use in nucleic acid separation from soil, environmental water, plant, or animal excrement.

9.  Use of the magnetic particle composition (e) according to any one of claims 1 to 8 in nucleic acid separation from soil, environmental water, plant, or animal excrement.

10. A kit (K) for obtaining a magnetic particle composition (e), comprising:

    a combination of magnetic particles (c) and a chaotropic salt (D), the magnetic particles (c) each including a core particle (P) that is a magnetic silica particle containing a magnetic metal oxide particle (A),
    wherein the magnetic metal oxide particle (A) in the core particle (P) has a weight percentage of 60 wt% or more based on the weight of the core particle (P),
    the magnetic particles (c) have a particle size distribution with a coefficient of variation of 5 to 50%, and
    the magnetic particle composition (e) can be obtained by mixing the magnetic particles (c) and the chaotropic salt (D).

11. Magnetic particles (c) for obtaining the magnetic particle composition (e) according to any one of claims 1 to 8 or the kit (K) according to claim 10,

    wherein the magnetic particles (c) each include a core particle (P) that is a magnetic silica particle containing a magnetic metal oxide particle (A),
    the magnetic metal oxide particle (A) in the core particle (P) has a weight percentage of 60 wt% or more based on the weight of the core particle (P), and
the magnetic particles (c) have a particle size distribution with a coefficient of variation of 5 to 50%.

**12.** A chaotropic salt (D) for obtaining the magnetic particle composition (e) according to any one of claims 1 to 8 or the kit (K) according to claim 10.

**13.** A separation and purification method of separating a substance to be separated (G) from a sample (F) using the magnetic particle composition (e) according to any one of claims 1 to 8.

**14.** The separation and purification method according to claim 13,
wherein the sample (F) is soil, environmental water, plant, or animal excrement, and the substance to be separated (G) is a nucleic acid.

**15.** The separation and purification method according to claim 13 or 14,

wherein the substance to be separated (G) is a target substance (G1),
the method comprising:

a composite formation step of forming a composite (H1) of the magnetic particles (c) and the target substance (G1) by contacting a sample (F1) containing the target substance (G1) with the magnetic particle composition (e) according to any one of claims 1 to 8;
a composite separation step of separating the composite (H1) from the sample (F1) by a magnetic force; and
a target substance dissociation step of obtaining the target substance (G1) from the composite (H1) by adding a dissociation solution (I).

**16.** The separation and purification method according to claim 15,
wherein in the composite formation step, the sample (F1) containing the target substance (G1) is contacted with the magnetic particle composition (e) in the presence of ethanol.

**17.** The separation and purification method according to claim 13 or 14,

wherein the substance to be separated (G) is a non-target substance (G2),
the method comprising:
a composite formation step of forming a composite (H2) of the magnetic particles (c) and the non-target substance (G2) by contacting a sample (F2) containing the target substance (G1) and the non-target substance (G2) with the magnetic particle composition (e) according to any one of claims 1 to 8; and
a non-target substance removal step of removing the non-target substance (G2) from the sample (F2) by separating the composite (H2) from the sample (F2) by a magnetic force to obtain a sample (F3) containing the target substance (G1).

**18.** The separation and purification method according to claim 17,
wherein the non-target substance (G2) comprises multiple types of non-target substances.

**19.** The separation and purification method according to any one of claims 13 to 18,
wherein the substance to be separated (G) is at least one selected from the group consisting of DNA and RNA.

## INTERNATIONAL SEARCH REPORT

| International application No. |
| --- |
| PCT/JP2020/034009 |

### A. CLASSIFICATION OF SUBJECT MATTER

C01B 33/12(2006.01)i; B03C 1/00(2006.01)i; B03C 1/01(2006.01)i; C01B 33/18(2006.01)i; C12N 15/10(2006.01)i; G01N 33/552(2006.01)i
FI:      C01B33/12 A; B03C1/00 A; B03C1/00 H; B03C1/01; C01B33/18 C; C12N15/10 114Z; G01N33/552

According to International Patent Classification (IPC) or to both national classification and IPC

### B. FIELDS SEARCHED

Minimum documentation searched (classification system followed by classification symbols)
C01B33/12; B03C1/00; B03C1/01; C01B33/18; C12N15/10; G01N33/552

Documentation searched other than minimum documentation to the extent that such documents are included in the fields searched
Published examined utility model applications of Japan        1922-1996
Published unexamined utility model applications of Japan      1971-2020
Registered utility model specifications of Japan              1996-2020
Published registered utility model applications of Japan      1994-2020

Electronic data base consulted during the international search (name of data base and, where practicable, search terms used)
JSTPlus/JST7580/JSTChina (JDreamIII)

### C. DOCUMENTS CONSIDERED TO BE RELEVANT

| Category* | Citation of document, with indication, where appropriate, of the relevant passages | Relevant to claim No. |
| --- | --- | --- |
| X<br>Y | Materials Letters, 2012, vol. 67, pp. 379-382, abstract, 2.1, 2.3, 3., fig. 1 | 1-6, 8-14, 19<br>15-18 |
| X | JP 2018-151384 A (SANYO CHEMICAL INDUSTRIES, LTD.) 27 September 2018 (2018-09-27) claim 3, tables 1, 2 | 11 |
| X | JP 2016-158558 A (JSR CORPORATION) 05 September 2016 (2016-09-05) paragraph [0024] | 12 |
| Y | JP 2016-90570 A (SANYO CHEMICAL INDUSTRIES, LTD.) 23 May 2016 (2016-05-23) paragraphs [0008], [0043]-[0045] | 15-18 |
| A | Journal of the American Chemical Society, 2006, vol. 128, pp. 15582-15583, entire text, all drawings | 1-19 |

☐ Further documents are listed in the continuation of Box C.      ☒ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 02 November 2020 (02.11.2020) | 17 November 2020 (17.11.2020) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
| --- | --- |

Form PCT/ISA/210 (second sheet) (January 2015)

## INTERNATIONAL SEARCH REPORT
Information on patent family members

| International application no. |
| --- |
| PCT/JP2020/034009 |

| Patent Documents referred in the Report | Publication Date | Patent Family | Publication Date |
| --- | --- | --- | --- |
| JP 2018-151384 A | 27 Sep. 2018 | (Family: none) | |
| JP 2016-158558 A | 05 Sep. 2016 | (Family: none) | |
| JP 2016-90570 A | 23 May 2016 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019170710 A

**Non-patent literature cited in the description**

- *Procedia Environmental Sciences,* 2013, vol. 18, 856-863
- **R. MASSART.** *IEEE Trans. Magn.,* 1981, vol. 17, 1247